(19) Europäisches Patentamt / European Patent Office / Office européen des brevets

(11) **EP 1 622 677 B1**

(12) **EUROPEAN PATENT SPECIFICATION**

(45) Date of publication and mention
of the grant of the patent:
**18.09.2013 Bulletin 2013/38**

(21) Application number: **04749543.7**

(22) Date of filing: **31.03.2004**

(51) Int Cl.:
*A61N 1/08* (2006.01)     *A61N 1/05* (2006.01)
*G01R 33/28* (2006.01)

(86) International application number:
**PCT/US2004/009754**

(87) International publication number:
**WO 2004/095385 (04.11.2004 Gazette 2004/45)**

(54) **DEVICE FOR PREVENTING MAGNETIC-DEVICE IMAGING INDUCED DAMAGE**

EINRICHTUNG ZUR VERHINDERUNG VON DURCH MAGNETEINRICHTUNGSABBILDUNG VERURSACHTEN BESCHÄDIGUNGEN

DISPOSITIF PERMETTANT D'EMPECHER LES DOMMAGES DUS A L'IMAGERIE PAR RESONANCE MAGNETIQUE

(84) Designated Contracting States:
**AT BE BG CH CY CZ DE DK EE ES FI FR GB GR HU IE IT LI LU MC NL PL PT RO SE SI SK TR**

(30) Priority: **02.04.2003 US 405154
08.03.2004 US 795746
08.03.2004 US 795744
08.03.2004 US 795747
08.03.2004 US 795742
08.03.2004 US 795743
08.03.2004 US 795741**

(43) Date of publication of application:
**08.02.2006 Bulletin 2006/06**

(73) Proprietor: **Medtronic, Inc.
Minneapolis, MN 55432-5604 (US)**

(72) Inventors:
• **WEINER, Michael, L.
Webster, NY 14580 (US)**

• **CONNELLY, Patrick, R.
Rochester, NY 14620 (US)**
• **HELFER, Jeffrey, L.
Victor, NY 14564 (US)**
• **MILLER, Victor
Clarence, NY 14031 (US)**

(74) Representative: **Hughes, Andrea Michelle et al
Dehns
St Bride's House
10 Salisbury Square
London EC4Y 8JD (GB)**

(56) References cited:
**US-A- 5 217 010     US-A- 5 523 534
US-A- 5 523 534     US-A- 5 699 801
US-A- 5 827 997     US-A- 5 837 940
US-A- 5 916 161     US-A- 6 134 478
US-A1- 2002 133 086**

## Description

## TECHNICAL FIELD

The present invention relates generally to a device and method for preventing magnetic-resonance imaging induced damage. More particularly, the present invention is directed to medical assist systems, which may include leads and other implantable or non-implantable components, that are shielded by segmented shielding to hardened or immune the systems from electromagnetic interference or insult, namely electromagnetic interference or insult in a magnetic-resonance imaging environment and to a modifiable magnetic-resonance imaging, which is, automatically or manually, responsive to sensed tissue temperature changes or known localized specific energy absorption ratios.

## BACKGROUND ART

Magnetic- resonance imaging ("magnetic- resonance imaging") has been developed as an imaging technique adapted to obtain both images of anatomical features of human patients as well as some aspects of the functional activities of biological tissue. These images have medical diagnostic value in determining the state of the health of the tissue examined.

In a magnetic-resonance imaging process, a patient is typically aligned to place the portion of the patient's anatomy to be examined in the imaging volume of the magnetic-resonance imaging apparatus. Such an magnetic-resonance imaging apparatus typically comprises a primary magnet for supplying a constant magnetic field ($B_0$) which, by convention, is along the z-axis and is substantially homogeneous over the imaging volume and secondary magnets that can provide linear magnetic field gradients along each of three principal Cartesian axes in space (generally x, y, and z, or $x_1$, $x_2$ and $x_3$, respectively). The apparatus also comprises one or more radio-frequency coils that provide excitation and detection of the magnetic-resonance imaging signal.

The use of the magnetic-resonance imaging process with patients who have implanted or non-implanted medical assist devices; such as, but not limited to, cardiac assist devices, implanted insulin pumps, catheter guide wires, leads for neurostimulation probes, intraluminal coils, guided catheters, temporary cardiac pacemakers, temporary esophageal pacemakers; often presents problems.

For a specific example, as is known to those skilled in the art, implantable devices (such as implantable pulse generators (IPGs) and cardioverter/ defibrillator/pacemakers (CDPs)) are sensitive to a variety of forms of electromagnetic interference (EMI) because these enumerated devices include sensing and logic systems that respond to low-level electrical signals emanating from the monitored tissue region of the patient and that these devices may also have metal wire leads, which can act as antenna and provide a path for the induced energy to travel to and possibly damage power sensitive circuitry.

Since the sensing systems and conductive elements of these medical assist devices are responsive to changes in local electromagnetic fields, the medical assist devices are vulnerable to external sources of severe electromagnetic noise, and in particular, to electromagnetic fields emitted during the magnetic-resonance imaging (magnetic-resonance imaging) procedure. Thus, patients with medical assist devices are generally advised not to undergo magnetic-resonance imaging (magnetic-resonance imaging) procedures.

To more appreciate the problem using a specific illustration, namely the use of implantable cardiac assist devices during a magnetic-resonance imaging process, will be briefly discussed.

The human heart may suffer from two classes of rhythmic disorders or arrhythmias: bradycardia and tachyarrhythmia. Bradycardia occurs when the heart beats too slowly, and may be treated by a common implantable pacemaker delivering low voltage (about 3 V) pacing pulses.

The common implantable pacemaker is usually contained within a hermetically sealed enclosure, in order to protect the operational components of the device from the aqueous environment of the body, as well as to protect the body from the device.

The common implantable pacemaker operates in conjunction with one or more electrically conductive leads, adapted to conduct electrical stimulating pulses to sites within the patient's heart and to communicate sensed signals from those sites back to the implanted device.

Furthermore, the common implantable pacemaker typically has a metal case and a connector block mounted to the metal case that includes receptacles for leads which may be used for electrical stimulation or which may be used for sensing of physiological signals. The battery and the circuitry associated with the common implantable pacemaker are hermetically sealed within the case. Electrical interfaces are employed to connect the leads outside the metal case with the medical device circuitry and the battery inside the metal case.

Electrical interfaces serve the purpose of providing an electrical circuit path extending from the interior of a hermetically sealed metal case to an external point outside the case while maintaining the hermetic seal of the case. A conductive path is provided through the interface by a conductive pin that is electrically insulated from the case itself.

Such interfaces typically include a ferrule that permits attachment of the interface to the case, the conductive pin, and a hermetic glass or ceramic seal that supports the pin within the ferrule and isolates the pin from the metal case.

A common implantable pacemaker can, under some circumstances, be susceptible to electrical interference such that the desired functionality of the pacemaker is impaired. For example, common implantable pacemaker requires protection against electrical interference from electromagnetic interference (EMI), defibrillation pulses, electrostatic discharge, or other generally large voltages or currents generated by other devices external to the medical device. As noted above, more recently, it has become crucial that cardiac assist systems be protected from magnetic-resonance imaging sources.

Such electrical interference can damage the circuitry of the cardiac assist systems or cause interference in the proper operation or functionality of the cardiac assist systems. For example, damage may occur due to high voltages or excessive currents introduced into the cardiac assist system by voltages or currents induced in the cardiac assist system circuitry or on the wire leads leading to and from the cardiac assist system circuitry.

Therefore, it is required that such voltages and currents be limited at the input of such cardiac assist systems, e.g., at the interface. Protection from such voltages and currents has typically been provided at the input of a cardiac assist system by the use of one or more zener diodes and one or more filter capacitors.

For example, one or more zener diodes may be connected between the circuitry to be protected, e.g., pacemaker circuitry, and the metal case of the medical device in a manner which grounds voltage surges and current surges through the diode(s). Such zener diodes and capacitors used for such applications may be in the form of discrete components mounted relative to circuitry at the input of a connector block where various leads are connected to the implantable medical device, e.g., at the interfaces for such leads.

 However, such protection, provided by zener diodes and capacitors placed at the input of the medical device, increases the congestion of the medical device circuits, at least one zener diode and one capacitor per input/ output connection or interface. This is contrary to the desire for increased miniaturization of implantable medical devices.

Further, when such protection is provided, interconnect wire length for connecting such protection circuitry and pins of the interfaces to the medical device circuitry that performs desired functions for the medical device tends to be undesirably long. The excessive wire length may lead to signal loss and undesirable inductive effects. The wire length can also act as an antenna that conducts undesirable electrical interference signals to sensitive CMOS circuits within the medical device to be protected.

Additionally, the radio frequency (radio- frequency) energy that is inductively coupled into the wire causes intense heating along the length of the wire, and at the electrodes that are attached to the heart wall. This heating may be sufficient to ablate the interior surface of the blood vessel through which the wire lead is placed, and may be sufficient to cause scarring at the point where the electrodes contact the heart. A further result of this ablation and scarring is that the sensitive node that the electrode is intended to pace with low voltage signals becomes desensitized, so that pacing the patient's heart becomes less reliable, and in some cases fails altogether. Additionally, the switching of the gradient magnetic fields may also induce unwanted voltages causing problems with the circuitry and potential pacing of the heart.

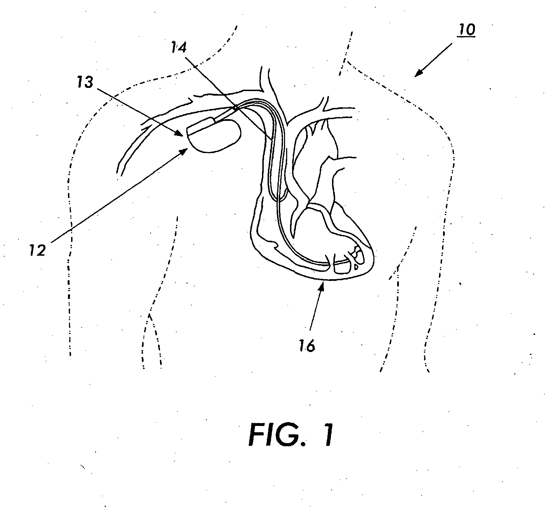

Another conventional solution for protecting the implantable medical device from electromagnetic interference is illustrated in Figure 1. Figure 1 is a schematic view of an implantable medical device **12** embodying protection against electrical interference. At least one lead **14** is connected to the implantable medical device **12** in connector block region **13** using an interface.

In the case where implantable medical device **12** is a pacemaker implanted in a body **10**, the pacemaker **12** includes at least one or both of pacing and sensing leads represented generally as leads **14** to sense electrical signals attendant to the depolarization and repolarization of the heart **16**, and to provide pacing pulses for causing depolarization of cardiac tissue in the vicinity of the distal ends thereof.

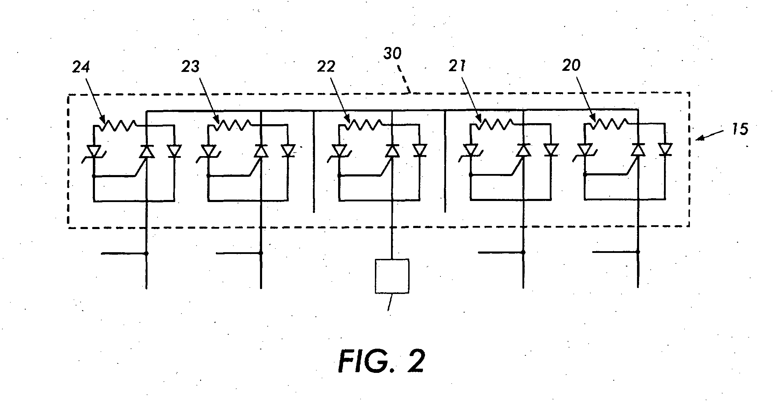

Figure 2 more particularly illustrates the circuit that is used conventionally to protect from electromagnetic interference. As shown in Figure 2, protection circuitry **15** is provided using a diode array component **30**. The diode array consists of five zener diode triggered semiconductor controlled rectifiers (SCRs) with anti-parallel diodes arranged in an array with one common connection. This allows for a small footprint despite the large currents that may be carried through the device during defibrillation, e.g., 10 amps. The SCRs **20-24** turn on and limit the voltage across the device when excessive voltage and current surges occur.

Some of the zener diode triggered SCRs may be connected to an electrically conductive pin, with each electrically conductive pin being connected to a medical device contact region to be wire bonded to pads of a printed circuit board. The diode array component **30** of Figure 2 may be connected to the electrically conductive pins via die contact regions along with other electrical conductive traces of the printed circuit board

Other attempts have been made to protect medical assist devices from magnetic-resonance imaging fields. For example, United States Patent 5,968,083 (to Ciciarelli et al.) describes a device adapted to switch between low and high impedance modes of operation in response to EMI insult. Furthermore, United States Patent 6,188,926 (to Vock) discloses a control unit for adjusting a cardiac pacing rate of a pacing unit to an interference backup rate when heart activity cannot be sensed due to EMI.

Another problem associated with magnetic-resonance imaging is the temperature change in tissue regions caused by using conventional magnetic-resonance imaging techniques. When a substance such as human tissue is subjected to a static magnetic field, the individual magnetic moments of the spins in the tissue align in a parallel and anti-parallel direction with the static magnetic field. This direction along the static magnetic field can be termed as the longitudinal direction. In magnetic-resonance imaging, the radio frequency polarizing field used for spin manipulation is constantly changing and thus, the individual magnetic moments of the spins in the tissue attempt to align with the polarizing field. The constant changing of alignment of the magnetic moments of the spins in the tissue causes the tissue's temperature to increase, thereby exposing the tissue to possible magnetic-resonance imaging induced thermal damage.

Although, conventional medical assist devices provide some means for protection against electromagnetic interference, these conventional medical assist devices require much circuitry and fail to provide fail-safe protection against radiation produced by magnetic-resonance imaging procedures. Moreover, the conventional medical assist devices fail to address the possible damage that can be done at the tissue interface due to radio-frequency-induced heating. Furthermore, the conventional medical assist devices fail to address the unwanted tissue region stimulation that may result from radio-frequency-induced electrical currents. Lastly, conventional magnetic-resonance imaging processes fail to provide a proper safeguard against potential magnetic-resonance imaging induced thermal damage due to the tissue's exposure to the switching magnetic field gradients and the circularly polarized Radio Frequency Field of the magnetic-resonance imaging process.

Thus, it is desirable to provide protection against electromagnetic interference, without requiring much circuitry and to provide fail-safe protection against radiation produced by magnetic-resonance imaging procedures. Moreover, it is desirable to provide medical assist devices that prevent the possible tissue damage that can be done at the tissue interface due to induced electrical signals. Furthermore, it is desirable to provide an effective means for transferring energy from one point in the body to another point without having the energy causing a detrimental effect upon the body. Lastly, it is desirable to implement a magnetic-resonance imaging process, which can be modified, automatically or manually, in response to sensed tissue temperature changes or known localized specific energy absorption rates, so as to prevent possible magnetic-resonance imaging induced thermal damage.

The patent document US 5,523,534 discloses the preamble of claim 1.

## DISCLOSURE OF INVENTION

The present invention provides a lead system as defined in independent claim 1.

## BRIEF DESCRIPTION OF DRAWINGS

The present invention may take form in various components and arrangements of components, and in various steps and arrangements of steps. The drawings are only for purposes of illustrating a preferred embodiment and are not to be construed as limiting the present invention, wherein:

Figures 1 and 2 are illustrations of conventional techniques used to protect against electromagnetic interference;

Figure 3 is a cross-sectional view of one embodiment of segmented shielding of a wire lead according to the concepts of the present invention;

Figure 4 is a cross-sectional view of another arrangement of segmented shielding of a wire lead not according to the concepts of the present invention;

Figure 5 is a top view of a layer of an arrangement of the shielding, having a predetermined segmented pattern of conductive materials, for a wire lead shielding not according to the concepts of the present invention;

Figure 6 is a top view of a layer of another arrangement of the shielding, having a predetermined segmented pattern of conductive materials, for a wire lead not according to the concepts of the present invention;

Figure 7 is a top view of a layer of another arrangement of the shielding, having a random pattern of conductive materials, for a wire lead not according to the concepts of the present invention;

Figure 8 is a flowchart illustrating the altering of a magnetic-resonance imaging acquisition process in response to a measured temperature of the tissue region being imaged, according to the concepts of the present invention;

Figure 9 is a flowchart illustrating the altering of a magnetic-resonance imaging acquisition process in response to a measured localized specific absorption ratio of the tissue region being imaged, according to the concepts of the present invention;

Figure 10 is a block diagram of an ECG amplifier capable of operating in the high static magnetic field, radio-frequency field, and gradient field environment produced in a magnetic-resonance imaging system, according to the concepts of the present invention;

Figure 11 is a block diagram of a pacemaker capable of operating in the high static magnetic fields, radio-frequency field, and gradient field environment produced in a magnetic-resonance imaging system, according to the concepts of the present invention;

Figure 12 is a block diagram of an implantable pacemaker, designed for external programming, which operates safely in the environment produced in a magnetic-resonance imaging system, according to the concepts of the present invention;

Figures 13 through 18 are schematic drawings of various ECG lead and/or harness configurations designed for operation in the environment produced by an magnetic-resonance imaging system, according to the concepts of the present invention;

Figures 19 and 20 are block diagrams of ECG amplifiers with a one-stage radio-frequency filter design, not according to the concepts of the present invention, for use with dual unshielded leads;

Figures 21 and 22 are block diagrams of ECG amplifiers with a one-stage radio-frequency filter design, according to the concepts of the present invention, for use with dual shielded leads;

Figures 23 and 24 are block diagrams of ECG amplifiers with a multistage radio-frequency filter design, not according to the concepts of the present invention, for use with dual unshielded leads;

Figures 25 and 26 are block diagrams of ECG amplifiers with a multistage radio-frequency filter design, according to the concepts of the present invention, for use with dual shielded leads;

Figures 27 and 28 are block diagrams of ECG amplifiers with a multistage radio-frequency filter design, according to the concepts of the present invention, for use with a multi-lead ECG harness;

Figure 29 is a block diagram of a magnetic-resonance imaging safe implantable pacemaker in accordance with the present invention;

Figure 30 is a block diagram of a magnetic-resonance imaging safe implantable pacemaker using a multistage radio-frequency filter, according to the concepts of the - present invention;

Figure 31 is a block diagram of an implantable stimulator capable of operating in a magnetic-resonance imaging system, in accordance with the present invention;

Figure 32 is a cross-sectional view of an apertured shielding of a wire lead according to the concepts of the present invention;

Figure 33 is a cross-sectional view of an apertured shielding of a wire lead not according to the concepts of the present invention;

Figure 34 is a top view of a layer of apertured shielding, having a predetermined apertured pattern of non-conductive materials, for a wire lead shielding not according to the concepts of the present invention;

Figure 35 is a top view of a layer of apertured shielding, having a predetermined apertured pattern of non-conductive materials, for a wire lead not according to the concepts of the present invention; and

Figure 36 is a top view of a layer of the apertured shielding, having a random apertured pattern of non-conductive materials, for a wire lead not according to the concepts of the present invention.

## BESTMODE FOR CARRYING OUT THE INVENTION

As noted above, the present invention is directed to an implantable device, such as a medical assist device, that is immune or hardened to electromagnetic insult or interference.

For a general understanding of the present invention, reference is made to the drawings. In the drawings, like reference have been used throughout to designate identical elements. In describing the present invention, the following term(s) have been used in the description.

For the purposes of the description below and the appended claims, the term, medical assist device/system or tissue invasive device/system, refers to any device/system that may enable monitoring of living tissue (s) or living system(s) wherein the monitoring may be, but not limited to an EKG signal, an ECG signal, a glucose level, hormone level, cholesterol level, or magnetic resonance image. The medical assist device/system or tissue invasive device/system may also enable stimulus intervention to provide assistance to living tissue(s) or living system(s) so that the stimulus causes the selected body tissue or system to function as desired. The stimulus may be, but not limited to, a cardiac stimulating substance or electrical pulse, a blood thinning substance, insulin, estrogen, progesterone, or testosterone.

The medical assist device/system or tissue invasive device/system may also provide therapeutic treatment to the living tissue(s) or living system(s) so that the treatment causes the selected body tissue or system to function as desired. The stimulus may be, but not limited to, radio frequency or laser ablation.

Furthermore, the medical assist device/ system or tissue invasive device/ system may be implanted in a body cavity of a living organism, either temporarily or permanently, or subcutaneously implanted into a living organism either temporarily or permanently. Moreover, the medical assist device/ system or tissue invasive device/ system may be located external to the living organism. Examples of medical assist devices/ systems or tissue invasive devices/ systems are, but not limited to, wearable or implantable cardiac pacers (such as pacemakers) , implantable pulse generators (IPGs) , cardioverter/ defibrillator/ pacemakers (CDPs) , cardiac monitoring systems, insulin pump controllers, brain monitoring systems, cardiac assist devices, implanted insulin pumps, catheter guide wires, leads for neurostimulation probes, intraluminal coils, guided catheters, temporary cardiac pacemakers, temporary esophageal pacemakers, etc.

Although the thrust of the description of one embodiment of the present invention, below, will use a cardiac assist system as an example of the workings of the concepts of the present invention, the concepts are directed to any medical assist device/system, tissue invasive device/system or tissue interactive device/system which includes electrical components that require shielding from electromagnetic interference or insult such that the interference or insult could cause damage to the electrical components or damage to the surrounding tissue.

As noted above, a typical medical device may be a pacemaker. The pacemaker may include at least one or both of pacing and sensing leads represented generally as leads to sense electrical signals attendant to the depolarization and repolarization of the heart and to provide pacing pulses for causing depolarization of cardiac tissue in the vicinity of the distal ends thereof.

The pacemaker or cardiac assist device may include a primary device housing. The primary device housing may include a control circuit, such as a microprocessor integrated circuit for controlling the operations of the cardiac assist system. The control circuit may select a mode of operation for the cardiac assist system based on predetermined sensed parameters. The primary device housing may also include circuitry to detect and isolate cross talk between device pulsing operations and device sensing operations. The control circuit may isolate physiological signals using a noise filtering circuit or a digital noise filtering.

The control circuit can be programmable from a source external of the primary device housing or the control circuit can provide physiological diagnostics to a source external of the primary device housing.

The primary device housing may include a power source. The power source may be a battery power source in combination with a battery power source measuring circuit. Further, the control circuit may automatically adjust a value for determining an elective replacement indication condition of a battery power source such that the value is automatically adjusted by the control circuit in response to a measured level of a state of the battery power source, the measured level generated by the battery power source measuring circuit connected to the battery power source.

As noted above, both the primary housing and the leads of the medical assist device/system require protection or shielding from electromagnetic interference or insult. The present invention provides a shielding that substantially prevents electromagnetic interference from damaging any, contained therein, electrical components or damage to the tissue surrounding the medical assist device/system's primary housing or leads. The shielding is a compilation of patterned layers having non-conductive materials and conductive materials contained in each patterned layer.

As is well known, conductive material provides a shield or block for electromagnetic radiation, especially radio frequency radiation ("radio-frequency") or magnetic radiation ("magnetic-resonance imaging") used in magnetic-resonance imaging so that the magnetic-resonance imaging radiation cannot penetrate the conductive materials to do damage to any electrical components located on the other side of the shield.

However, although conductive material may provide a block to the magnetic-resonance imaging radiation so as to prevent penetration of the magnetic-resonance imaging radiation, the blocking of the magnetic-resonance imaging radiation can cause eddy currents to be induced in the conductive material by the changing magnetic fields in the magnetic-resonance imaging radiation. The eddy currents may cause heating of the conductive material, thereby heating the enclosed electrical components so as to cause damage or heating the surrounding tissue so as to cause tissue damage.

To prevent the eddy currents from being induced to a level, which may cause damage from excessive heat, the conductive material in the shielding of the present invention is patterned, apertured, or segmented. More specifically, magnetic-resonance imaging radiation shields generally contain thin layers of conductive cladding sometimes separated by insulators or dielectrics. In the present invention, the conductive material in a layer is patterned, apertured, or segmented so that the conductive material is literally broken up to limit the build up of eddy currents induced by the changing magnetic fields produced during magnetic-resonance imaging.

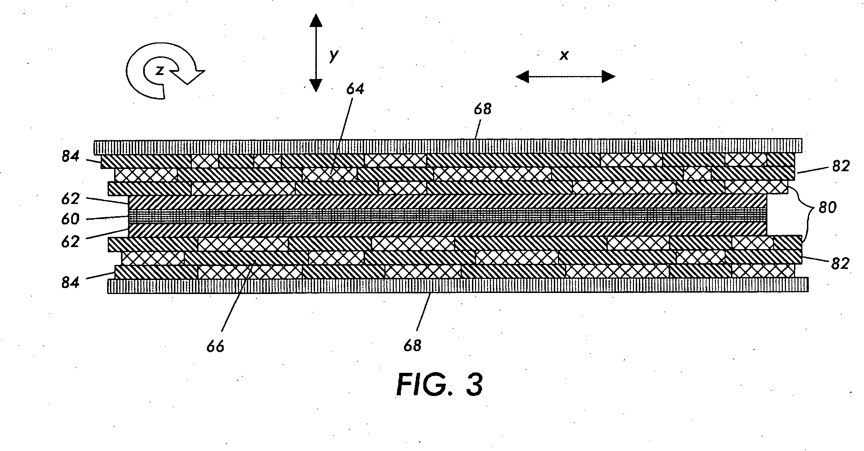

An example of such a patterned or segmented shielding for use with an electrical lead is illustrated in Figure 3. As shown in Figure 3, an electrical lead **60** is initially coated with an insulation layer **62** so as to electrically insulate the electrical lead from its surroundings. Upon the insulation layer **62**, a first patterned or segmented layer 80 of shielding is placed or formed thereon. The first patterned or segmented layer **80** includes conductive materials **64** and non-conductive material(s) **66**.

Upon the first patterned or segmented layer **80** of shielding, a second patterned or segmented layer **82** of shielding is placed or formed thereon. The second patterned or segmented layer **82** includes conductive materials **64** and non-conductive material(s) **66**.

It is noted that the non-conductive material 66 may be formed of a single integral piece of non-conductive material or be formed from a multitude of pieces of non-conductive material, the multitude of pieces being connected together in such a manner to function as a single integral piece of non-conductive material.

Upon the second patterned or segmented layer **82** of shielding, a third patterned or segmented layer **84** of shielding is placed or formed thereon. The third patterned or segmented layer **84** includes conductive materials **64** and non-conductive material(s) **66**.

The conductive materials **64** of this embodiment of the present invention may be a metal, a carbon composite, nanotubes (wherein the nanotubes may be constructed from a carbon base or the nanotubes could be formed from other amalgams coated with the appropriate material (s) ), metal-coated carbon filaments (wherein the metal may be one of the following metals: nickel, copper, cobalt, silver, gold, tin, or zinc), metal-coated ceramic filaments (wherein the metal may be one of the following metals: nickel, copper, cobalt, silver, gold, tin, or zinc), a composite of metal-coated carbon filaments and a polymer (wherein the polymer may be one of the following: polyether sulfone, silicone, polymide, polyvinylidene fluoride, or epoxy), a composite of metal-coated ceramic filaments and a polymer (wherein the polymer may be one of the following: polyether sulfone, silicone, polymide, polyvinylidene fluoride, or epoxy), a composite of metal-coated carbon filaments and a ceramic (wherein the ceramic may be one of the following: cement, silicates, phosphates, silicon carbide, silicon nitride, aluminum nitride, or titanium diboride) , a composite of metal-coated ceramic filaments and a ceramic (wherein the ceramic may be one of the following: cement, silicates, phosphates, silicon carbide, silicon nitride, aluminum nitride, or titanium diboride) , or a composite of metal- coated (carbon or ceramic) filaments (wherein the metal may be one of the following metals: nickel, copper, cobalt, silver, gold, tin, or zinc) and a polymer/ ceramic combination (wherein the polymer may be one of the following: polyether sulfone, silicone, polymide, polyvinylidene fluoride, or epoxy and the ceramic may be one of the following: cement, silicates, phosphates, silicon carbide, silicon nitride, aluminum nitride, or titanium diboride) .

A more detailed description of the coated filaments is found in US Patent Number 5,827,997, entitled "Metal Filaments for Electromagnetic Interference Shielding." The entire content of US Patent Number 5,827,997 is hereby incorporated by reference.

The non-conductive material(s) **66** of this embodiment of the present invention may be a ceramic; glass; mica; anodized copper; metallic oxides; natural or synthetic rubbers; or resins, such as natural resins, epoxy resins, or silicones.

Over the shielding, a biocompatible layer **68** may be placed or formed thereon. Preferably, the biocompatible layer is a non-permeable diffusion resistant biocompatible material.

As illustrated in Figure 3, the conductive materials **64** are segmented or patterned in an x-direction, wherein the x-direction is a direction substantially parallel to an axis of the lead **60**. In other words, the conductive materials **64** are broken up in this direction such that one conductive material **64** is physically separated from a neighboring conductive material **64** in the same layer.

Moreover, as illustrated in Figure 3, the conductive materials **64** of different layers are segmented or patterned in a y- direction, wherein the y- direction is a direction substantially perpendicular to an axis of the lead **60**. In other words, the conductive materials **64** of different layers are broken up in this direction such that one conductive material **64** is electrically isolated from a neighboring conductive material **64** in an immediate adjacent layer; i.e., a conductive material **64** in the first patterned or segmented layer **80** is electrically isolated from a neighboring conductive material **64** in the second patterned or segmented layer **82**.

Preferably, the x- directional gap between non co- layer immediately x- directional adjacent conductive materials **64** is much smaller than the wavelength of the electromagnetic pulse that the shield must attenuate to prevent the incident pulse from passing unattenuated through the shield.

Lastly, the conductive materials **64** may be segmented or patterned in a z- direction, wherein the z- direction represents the planar surface of a layer as it is wrapped around the lead **60**.

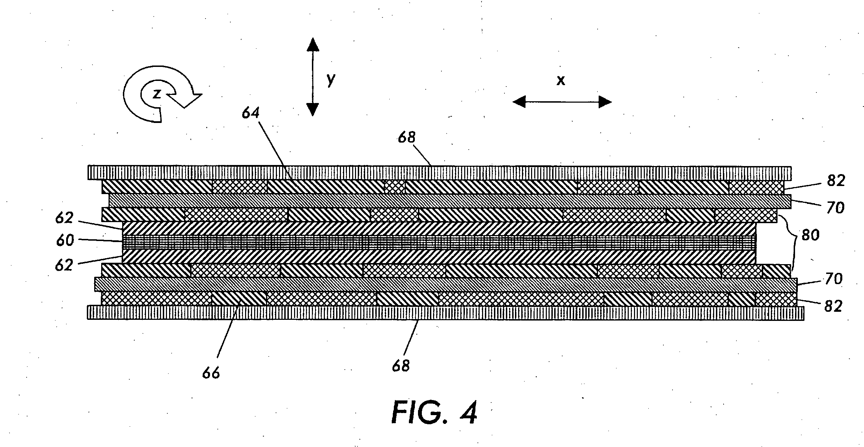

Another example of such a patterned or segmented shielding for use with an electrical lead is illustrated in Figure 4. As shown in Figure 4, an electrical lead **60** is initially coated with an insulation layer **62** so as to electrically insulate the electrical lead from its surroundings. Upon the insulation layer **62**, a first patterned or segmented layer **80** of shielding is placed or formed thereon. The first patterned or segmented layer **80** includes conductive materials **64** and non-conductive material(s) **66**.

As noted above, the non-conductive material **66** may be formed of a single integral piece of non-conductive material or be formed from a multitude of pieces of non-conductive material, the multitude of pieces being connected together in such a manner to function as a single integral piece of non-conductive material.

Upon the first patterned or segmented layer **80** of shielding, a layer **70** of non-conductive material is placed or formed thereon. Upon the layer **70** of non-conductive material, a second patterned or segmented layer **82** of shielding is placed or formed thereon. The second patterned or segmented layer **82** includes conductive materials **64** and non-conductive material(s) **66**.

The conductive materials **64** of this embodiment of the present invention may be a metal, a carbon composite, nanotubes (wherein the nanotubes may be constructed from a carbon base or the nanotubes could be formed from other amalgams coated with the appropriate material (s) ) , metal- coated carbon filaments (wherein the metal may be one of the following metals: nickel, copper, cobalt, silver, gold, tin, or zinc) , metal- coated ceramic filaments (wherein the metal may be one of the following metals: nickel, copper, cobalt, silver, gold, tin, or zinc) , a composite of metal- coated carbon filaments and a polymer (wherein the polymer may be one of the following: polyether sulfone, silicone, polymide, polyvinylidene fluoride, or epoxy) , a composite of metal- coated ceramic filaments and a polymer (wherein the polymer may be one of the following: polyether sulfone, silicone, polymide, polyvinylidene fluoride, or epoxy) , a composite of metal- coated carbon filaments and a ceramic (wherein the ceramic may be one of the following: cement, silicates, phosphates, silicon carbide, silicon nitride, aluminum nitride, or titanium diboride) , a composite of metal-coated ceramic filaments and a ceramic (wherein the ceramic may be one of the following: cement, silicates, phosphates, silicon carbide, silicon nitride, aluminum nitride, or titanium diboride) , or a composite of metal- coated (carbon or ceramic) filaments (wherein the metal may be one of the following metals: nickel, copper, cobalt, silver, gold, tin, or zinc) and a polymer/ ceramic combination (wherein the polymer may be one of the following: polyether sulfone, silicone, polymide, polyvinylidene fluoride, or epoxy and the ceramic may be one of the following: cement, silicates, phosphates, silicon carbide, silicon nitride, aluminum nitride, or titanium diboride) .

Over the shielding, a biocompatible layer 68 may be placed or formed thereon. Preferably, the biocompatible layer is a non-permeable diffusion resistant biocompatible material.

The non-conductive materials **66** of this embodiment of the present invention may be a ceramic; glass; mica; anodized copper; metallic oxides; natural or synthetic rubbers; or resins, such as natural resins, epoxy resins, or silicones.

As illustrated in Figure 4, the conductive materials **64** are segmented or patterned in an x-direction, wherein the x-direction is a direction substantially parallel to an axis of the lead **60**. In other words, the conductive materials **64** are broken up in this direction.

Moreover, as illustrated in Figure 4, the conductive materials **64** are patterned or segmented in a y- direction, wherein the y- direction is a direction substantially perpendicular to an axis of the lead **60**. In other words, the conductive materials **64** are broken up in this direction such that one conductive material **64** is electrically isolated from a neighboring conductive material **64** in an immediate adjacent layer; i.e., a conductive material **64** in the first patterned or segmented layer **80** is electrically isolated from a neighboring conductive material **64** in the second patterned or segmented layer **82**.

Preferably, the x- directional gap between non co- layer immediately x- directional adjacent conductive materials **64** is much smaller than the wavelength of the electromagnetic pulse that the shield must attenuate to prevent the incident pulse from passing unattenuated through the shield.

Lastly, the conductive materials **64** may be segmented in a z- direction, wherein the z- direction represents the planar surface of a layer as it is wrapped around the lead **60**.

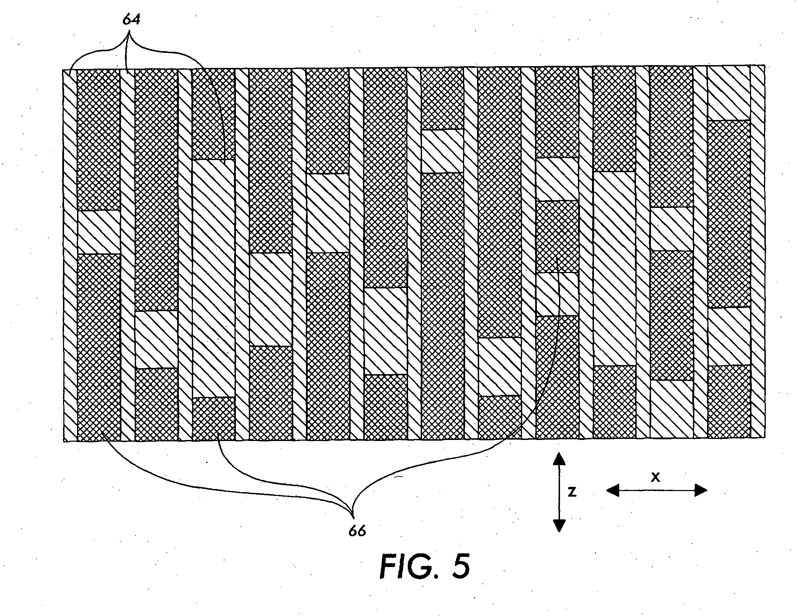

As noted above, the conductive material **64** is patterned or segmented to limit the build up of eddy currents. Examples of the patterning of the conductive materials **64** are illustrated in Figures 5-7.

Figure 5 illustrated a top view of a fabricated layer of the shielding. As illustrated in Figure 5, the conductive materials **64** are patterned or segmented in such a way that a width of the conductive material **64** in the x-direction, the x-direction being substantially parallel to an axis of a lead, is substantially equal. More specifically, the conductive materials **64** is patterned or segmented in such a way that conductive materials **64** spaced in a predetermined manner; e.g., the spacing between the conductive materials **64**, in the x-direction, may be equal or set in a predetermined manner. Lastly, the spacing between conductive materials **64** in a z-direction, the z-direction representing the planar surface of a layer as it is wrapped around a lead, is not necessarily equal or set in a predetermined manner; however the spacing in the z-direction may be equal or set in a predetermined manner.

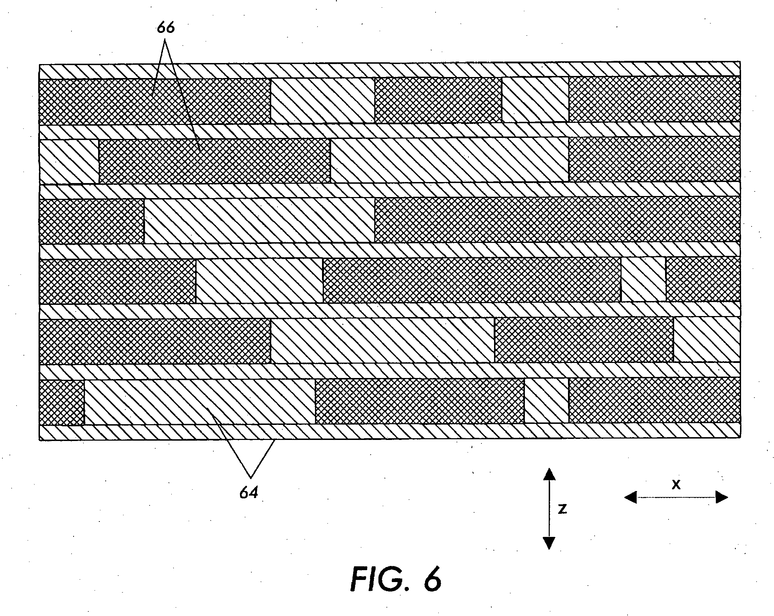

Figure 6 illustrated a top view of a fabricated layer of the shielding. As illustrated in Figure 6, the conductive materials **64** are patterned or segmented in such a way that a width of the conductive material **64** in the z- direction, the z-direction representing the planar surface of a layer as it is wrapped around a lead, is substantially equal. More specifically, the conductive materials **64** are patterned or segmented in such a way that conductive materials **64** spaced in a predetermined manner; e.g., the spacing between the conductive materials **64**, in the z-direction, may be equal or set in a predetermined manner. Lastly, the spacing between conductive materials **64** in a x-direction, the x-direction being substantially parallel to an axis of a lead, is not necessarily equal or set in a predetermined manner; however the spacing in the x-direction may be equal or set in a predetermined manner.

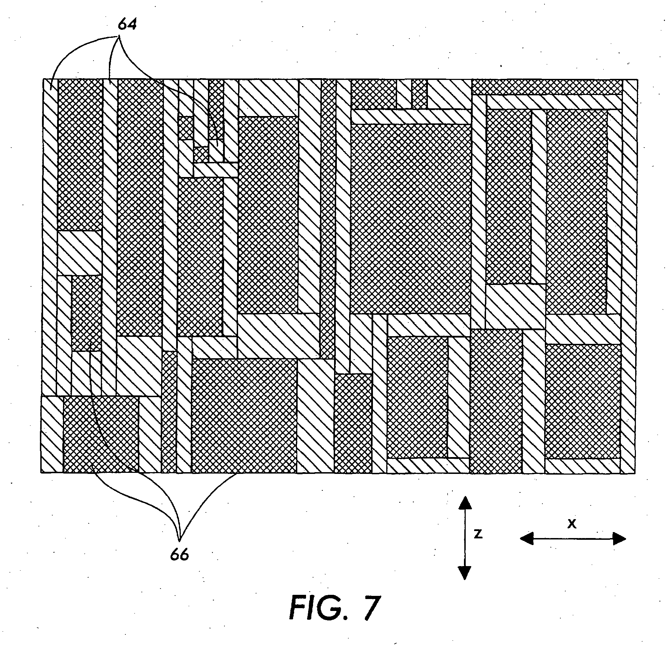

Figure 7 illustrated a top view of a fabricated layer of the shielding. As illustrated in Figure 7, the conductive material **64** are patterned or segmented in such a way that the widths of the conductive materials **64** in the z-direction, the z-direction representing the planar surface of a layer as it is wrapped around a lead, are random. More specifically, the conductive materials **64** are patterned or segmented in such a way that conductive materials **64** are spaced in a random manner. Lastly, the spacing between conductive materials **64** in an x-direction, the x-direction being substantially parallel to an axis of a lead, is also random.

It is noted that the although Figures 3-7 show a shielding with respect to a lead, the shielding can be used to shield other shaped devices such as electrical component housings or other components of a medical assist device/system.

It is further noted that the actual dimensions of the non-conductive materials can be set to suppress the radiation and eddy currents induced from specific frequencies; e.g., the lengths of the non-conductive material can be varied to suppress or block specific or undesired radiation frequencies. The dimensions of the non-conductive material would be on the order of the wavelength of the radiation so as to suppress, block, or shield the radiation.

The actual layers may be formed using a photoresist/masking process such as used in integrated circuit fabrication and thin film deposition. By using a mask/photoresist process, the conductive/insulative layers in the vertical direction can be easily formed so that the conductive/insulative layers alternate (radially outward from the axis of the lead), and the conductive/insulative layers in the horizontal direction also alternate (substantially parallel to an axis of the lead).

As noted above, the shielding may also be apertured. More specifically, implantable medical devices, such as pacemaker leads, typically demonstrate heating of 10-100 °C, whereas body tissues can be irreversibly damaged by increases in temperature of as little as 2-4 °C. Therefore, up to a 50X reduction in the amount of energy delivered to the implanted medical device is required to make the device safe, 100X if a reasonable design margin of safety is desired. This reduction in the amount of energy delivered to the implanted medical device corresponds to 20 dB of attenuation.

The performance of a shield or shield effectiveness (SE) with a circular opening has been shown to be:

$$SE \, (dB) \; = \; 20 \, Log \, [ \, \lambda / 2\pi r \, ]$$

where $\lambda$ is the incident wavelength and r is the radius of the circular opening.

For holes of other shapes (e.g. an annulus) the factor $2\pi r$ may be replaced by 2L where L is the largest dimension of the opening (i.e. the circumference):

$$SE \, (dB) \; = \; 20 \, Log \, [ \, \lambda / 2L \, ]$$

This formula indicates that a slot or annulus with a greatest dimension equal to 1/20 of the wavelength is required to provide a shielding effectiveness (SE) against heating effects of 20 dB.

The wavelength at a particular frequency in air is given by the equation $\lambda = c / f$, where c is the speed of light ($3 \times 10^8$ meters/second) and f is the frequency. However, the wavelength in a particular material, such as body tissues, is given by the following equation:

$$\lambda_m = \lambda_o / [ \, \varepsilon_{rel} \, ]^{1/2}$$

where $\varepsilon_{rel}$ is the relative dielectric constant.

At a frequency of 64 MHz and $\varepsilon_{rel} = 80$ (a typical value for body tissue), $\lambda_o$ is equal to 4.7 meters, and $\lambda_m$ is equal to 0.5 meters. At a frequency of 64 MHz, a shield effectiveness of 20 dB requires that L, the maximum aperture dimension (i.e. annulus circumference), not exceed 0.025 meters or 25 millimeters (L = 0.5 meters/20).

However, it is well known that shielding effectiveness is reduced as the number of openings N is increased. Shield effectiveness scales as the square root on N.

In the present invention, N may range from 10 to 1000. Therefore, to maintain minimum shield effectiveness against heating effects of 20 dB, the maximum aperture dimension should not exceed approximately 10 millimeters to 1 millimeter, depending upon how many apertures or openings are incorporated into the shield design. At higher frequencies such as 128 MHz (the frequency of emerging MRI facilities), the maximum aperture dimension should not exceed 5 millimeters to 0.5 millimeters.

Moreover, implantable medical devices, such as pacemaker leads, must also be capable of making sensitive electrical measurements such as intracardiac ECG signals, which are typically 1- 25 millivolts. To insure the accurate measurements of such signals, a measurement resolution capability of less than 0.1 millivolt is required. Experimental measurements have shown that MRI procedures can induce voltages of as much as 100 volts in implanted pacing leads. Therefore, a shield attenuation of 1, 000, 000 or 60 dB is required, which requires that the maximum aperture dimension must equal 1/2000 of the wavelength. At 64 MHz, L = 0.5 meters/ 2000 = 0.00025 meters or 0.25 millimeters. Since N may range from 10 to 1000, the maximum aperture dimension is further reduced to approximately 0.1 millimeters to 0.01 millimeters.

Therefore, depending upon the specific application and shield design, a maximum aperture dimension of 0.01 millimeters to 10 millimeters should be used.

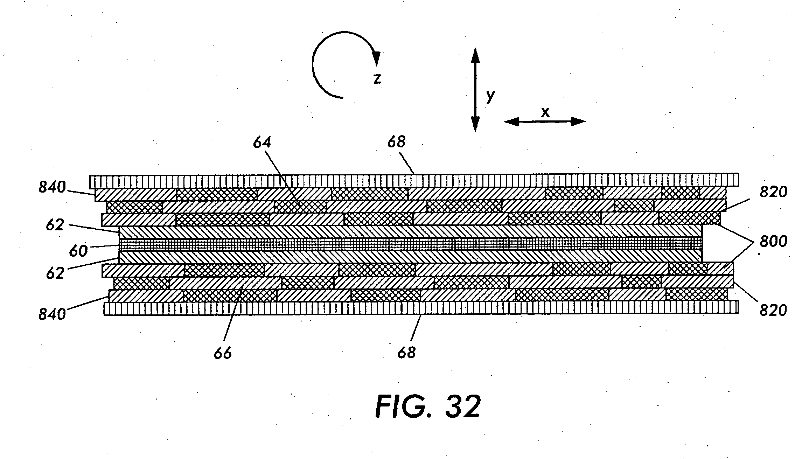

An example of such an apertured shielding for use with an electrical lead is illustrated in Figure 32. As shown in Figure 32, an electrical lead **60** is initially coated with an insulation layer **62** so as to electrically insulate the electrical lead from its surroundings. Upon the insulation layer **62**, a first patterned or apertured layer **800** of shielding is placed or formed thereon. The first patterned or apertured layer **800** includes conductive material **64** and non-conductive materials **66**.

Upon the first patterned or apertured layer **800** of shielding, a second patterned or apertured layer **820** of shielding is placed or formed thereon. The second patterned or apertured layer **820** includes conductive material **64** and non-conductive materials **66**.

It is noted that the conductive material **64** may be formed of a single integral piece of conductive material or be formed from a multitude of pieces of conductive material, the multitude of pieces being electrically connected together in such a manner to function as a single integral piece of conductive material.

Upon the second patterned or apertured layer **820** of shielding, a third patterned or apertured layer **840** of shielding is placed or formed thereon. The third patterned or apertured layer **840** includes conductive material **64** and non-conductive materials **66**.

The conductive materials **64** of this embodiment of the present invention may be a metal, a carbon composite, nanotubes (wherein the nanotubes may be constructed from a carbon base or the nanotubes could be formed from other amalgams coated with the appropriate material (s) ) , metal- coated carbon filaments (wherein the metal may be one of the following metals: nickel, copper, cobalt, silver, gold, tin, or zinc) , metal- coated ceramic filaments (wherein the metal may be one of the following metals: nickel, copper, cobalt, silver, gold, tin, or zinc) , a composite of metal- coated carbon filaments and a polymer (wherein the polymer may be one of the following: polyether sulfone, silicone, polymide, polyvinylidene fluoride, or epoxy) , a composite of metal- coated ceramic filaments and a polymer (wherein the polymer may be one of the following: polyether sulfone, silicone, polymide, polyvinylidene fluoride, or epoxy) , a composite of metal- coated carbon filaments and a ceramic (wherein the ceramic may be one of the following: cement, silicates, phosphates, silicon carbide, silicon nitride, aluminum nitride, or titanium diboride) , a composite of metal-coated ceramic filaments and a ceramic (wherein the ceramic may be one of the following: cement, silicates, phosphates, silicon carbide, silicon nitride, aluminum nitride, or titanium diboride) , or a composite of metal- coated (carbon or ceramic) filaments (wherein the metal may be one of the following metals: nickel, copper, cobalt, silver, gold, tin, or zinc) and a polymer/ ceramic combination (wherein the polymer may be one of the following: polyether sulfone, silicone, polymide, polyvinylidene fluoride, or epoxy and the ceramic may be one of the following: cement, silicates, phosphates, silicon carbide, silicon nitride, aluminum nitride, or titanium diboride) .

A more detailed description of the coated filaments is found in US Patent Number 5,827,997, entitled "Metal Filaments for Electromagnetic Interference Shielding." The entire content of US Patent Number 5,827,997 is hereby incorporated by reference.

The non-conductive materials **66** of this embodiment of the present invention may be a ceramic; glass; mica; anodized copper; metallic oxides; natural or synthetic rubbers; or resins, such as natural resins, epoxy resins, or silicones.

Over the shielding, a biocompatible layer **68** may be placed or formed thereon. Preferably, the biocompatible layer is a non-permeable diffusion resistant biocompatible material.

As illustrated in Figure 32, the conductive material **64** is patterned or apertured in an x-direction through the utilization of non-conductive materials **66**, wherein the x-direction is a direction substantially parallel to an axis of the lead 60. In other words, the conductive material **64** is broken up, patterned, or apertured in this direction such that one non-conductive material **66** is physically separated from a neighboring non-conductive material **66** in the same layer.

Moreover, as illustrated in Figure 32, the conductive materials **64** of different layers are patterned in a y- direction, wherein the y- direction is a direction substantially perpendicular to an axis of the lead **60**. In other words, the conductive materials **64** of different layers are broken up in this direction such that one conductive material **64** is electrically isolated from a neighboring conductive material **64** in an immediate adjacent layer; i.e., a conductive material **64** in the first patterned or apertured layer **800** is electrically isolated from a neighboring conductive material **64** in the second patterned or apertured layer **82**.

Preferably, the distance of the overlap is much smaller than the wavelength of the electromagnetic pulse that the shield must attenuate to prevent the incident pulse from passing unattenuated through the shield.

If the length of the overlapping shield dimension is designed to exceed the thickness of the shield material, the structure thus formed resembles a waveguide and frequencies well below its cut-off frequency are subject to additional attenuation. For a frequency of 64-128 MHz, this overlap corresponds to approximately 2-3X the maximum aperture dimension.

Therefore, depending upon the specific application and shield design, a minimum shield overlap dimension should be 2- 3X the maximum aperture dimension. It is also important to note that the heating may also increase with increased frequency. At a static magnetic field of 1.5 Tesla the RF transmission frequency is 63.7 MHz, at 3T it is 127.5 MHz (factor of 42.5 MHz for each Tesla) .

Lastly, the conductive material **64** may be segmented or apertured in a z- direction, wherein the z- direction represents the planar surface of a layer as it is wrapped around the lead **60**.

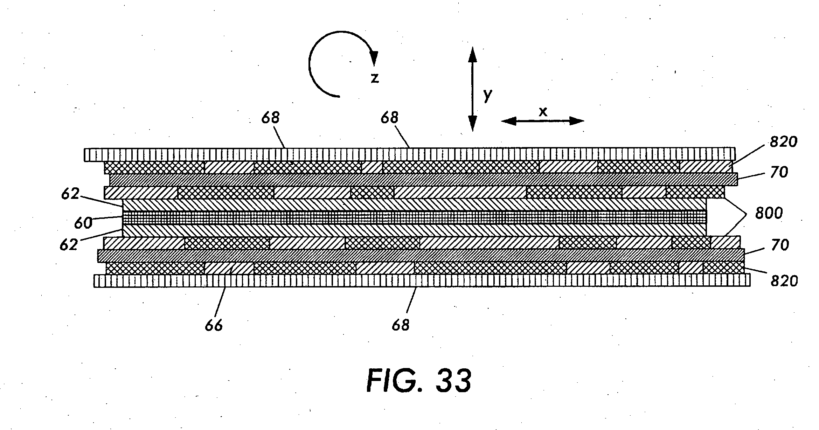

Another example of such a shielding for use with an electrical lead is illustrated in Figure 33. As shown in Figure 33, an electrical lead **60** is initially coated with an insulation layer **62** so as to electrically insulate the electrical lead from its surroundings. Upon the insulation layer **62**, a first patterned or apertured layer **800** of shielding is placed or formed thereon. The first patterned or apertured layer **800** includes conductive material **64** and non-conductive materials **66**.

It is noted that the conductive material **64** may be formed of a single integral piece of conductive material or be formed from a multitude of pieces of conductive material, the multitude of pieces being electrically connected together in such a manner to function as a single integral piece of conductive material.

Upon the first patterned or apertured layer **800** of shielding, a layer **70** of non-conductive material is placed or formed thereon. Upon the layer **70** of non-conductive material, a second patterned or apertured layer **820** of shielding is placed or formed thereon. The second patterned or apertured layer **820** includes conductive material **64** and non-conductive materials **66**.

The conductive materials **64** of this arrangement may be a metal, a carbon composite, nanotubes (wherein the nanotubes may be constructed from a carbon base or the nanotubes could be formed from other amalgams coated with the appropriate material (s) ) , metal- coated carbon filaments (wherein the metal may be one of the following metals: nickel, copper, cobalt, silver, gold, tin, or zinc) , metal- coated ceramic filaments (wherein the metal may be one of the following metals: nickel, copper, cobalt, silver, gold, tin, or zinc) , a composite of metal- coated carbon filaments and a polymer (wherein the polymer may be one of the following: polyether sulfone, silicone, polymide, polyvinylidene fluoride, or epoxy) , a composite of metal- coated ceramic filaments and a polymer (wherein the polymer may be one of the following: polyether sulfone, silicone, polymide, polyvinylidene fluoride, or epoxy) , a composite of metal-coated carbon filaments and a ceramic (wherein the ceramic may be one of the following: cement, silicates, phosphates, silicon carbide, silicon nitride, aluminum nitride, or titanium diboride) , a composite of metal- coated ceramic filaments and a ceramic (wherein the ceramic may be one of the following: cement, silicates, phosphates, silicon carbide, silicon nitride, aluminum nitride, or titanium diboride) , or a composite of metal- coated (carbon or ceramic) filaments (wherein the metal may be one of the following metals: nickel, copper, cobalt, silver, gold, tin, or zinc) and a polymer/ ceramic combination (wherein the polymer may be one of the following: polyether sulfone, silicone, polymide, polyvinylidene fluoride, or epoxy and the ceramic may be one of the following: cement, silicates, phosphates, silicon carbide, silicon nitride, aluminum nitride, or titanium diboride) .

Over the shielding, a biocompatible layer 68 may be placed or formed thereon. Preferably, the biocompatible layer is a non-permeable diffusion resistant biocompatible material.

The non-conductive materials 66 of this arrangement may be a ceramic; glass; mica; anodized copper; metallic oxides; natural or synthetic rubbers; or resins, such as natural resins, epoxy resins, or silicones.

As illustrated in Figure 4, the conductive materials **64** are patterned or apertured in an x-direction, wherein the x-direction is a direction substantially parallel to an axis of the lead **60**. In other words, the conductive materials **64** are broken up, patterned, or apertured in this direction such that the non-conductive materials **66** break up the conductive material **64**.

Moreover, as illustrated in Figure 33, the conductive materials **64** are patterned in a y- direction, wherein the y- direction is a direction substantially perpendicular to an axis of the lead **60**. In other words, the conductive materials **64** are broken up in this direction such that one conductive material **64** is electrically isolated from a neighboring conductive material **64** in an immediate adjacent layer; i.e., a conductive material **64** in the first patterned or apertured layer **800** is electrically isolated from a neighboring conductive material **64** in the second patterned or apertured layer **820**.

Preferably, the distance of the overlap is much smaller than the wavelength of the electromagnetic pulse that the shield must attenuate to prevent the incident pulse from passing unattenuated through the shield.

If the length of the overlapping shield dimension is designed to exceed the thickness of the shield material, the structure thus formed resembles a waveguide and frequencies well below its cut-off frequency are subject to additional attenuation. For a frequency of 64-128 MHz, this overlap corresponds to approximately 2-3X the maximum aperture dimension.

Therefore, depending upon the specific application and shield design, a minimum shield overlap dimension should be 2- 3X the maximum aperture dimension.

Lastly, the conductive materials **64** may be segmented in a z- direction, wherein the z- direction represents the planar surface of a layer as it is wrapped around the lead **60**.

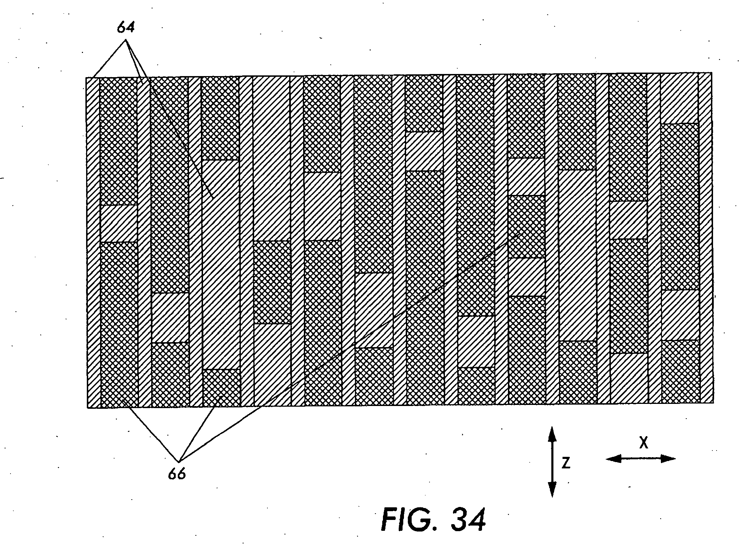

As noted above, the conductive material **64** is patterned or apertured to limit the build up of eddy currents. Examples of the patterning or aperturing of the conductive material **64** are illustrated in Figures 34-36.

Figure 34 illustrated a top view of a fabricated layer of the shielding. As illustrated in Figure 34, the conductive material **64** is patterned or apertured in such a way that a width of the conductive material **64** in the x-direction, the x-direction being substantially parallel to an axis of a lead, is substantially equal. More specifically, the conductive material **64** is patterned or apertured in such a way that non-conductive materials **66** spaced in a predetermined manner; e.g., the spacing between the non-conductive materials **66**, in the x-direction, may be equal or set in a predetermined manner. Lastly, the spacing between non-conductive materials **66** in a z-direction, the z-direction representing the planar surface of a layer as it is wrapped around a lead, is not necessarily equal or set in a predetermined manner; however the spacing in the z-direction may be equal or set in a predetermined manner.

Preferably, the distance of the overlap is much smaller than the wavelength of the electromagnetic pulse that the shield must attenuate to prevent the incident pulse from passing unattenuated through the shield.

If the length of the overlapping shield dimension is designed to exceed the thickness of the shield material, the structure thus formed resembles a waveguide and frequencies well below its cut-off frequency are subject to additional attenuation. For a frequency of 64-128 MHz, this overlap corresponds to approximately 2-3X the maximum aperture dimension.

Therefore, depending upon the specific application and shield design, a minimum shield overlap dimension should be 2- 3X the maximum aperture dimension.

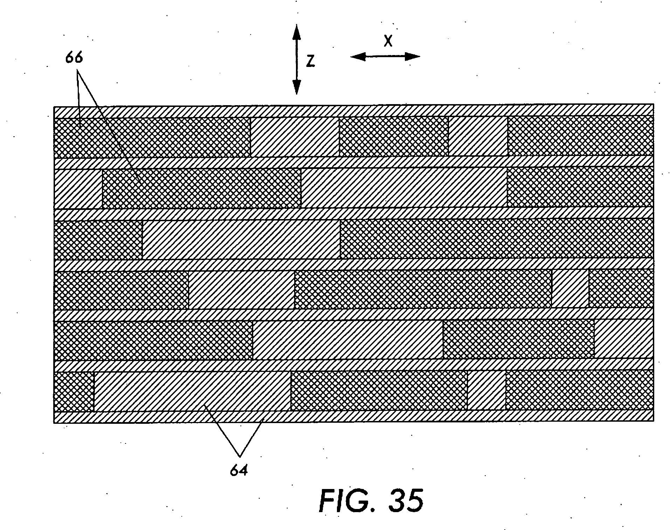

Figure 35 illustrated a top view of a fabricated layer of the shielding. As illustrated in Figure 35, the conductive material **64** is patterned or apertured in such a way that a width of the conductive material **64** in the z-direction, the z-direction representing the planar surface of a layer as it is wrapped around a lead, is substantially equal. More specifically, the conductive material **64** is patterned or apertured in such a way that non-conductive materials **66** spaced in a predetermined manner; e.g., the spacing between the non-conductive materials **66**, in the z-direction, may be equal or set in a predetermined manner. Lastly, the spacing between non-conductive materials 66 in a x-direction, the x-direction being substantially parallel to an axis of a lead, is not necessarily equal or set in a predetermined manner; however the spacing in the x-direction may be equal or set in a predetermined manner.

Preferably, the distance of the overlap is much smaller than the wavelength of the electromagnetic pulse that the shield must attenuate to prevent the incident pulse from passing unattenuated through the shield.

If the length of the overlapping shield dimension is designed to exceed the thickness of the shield material, the structure thus formed resembles a waveguide and frequencies well below its cut-off frequency are subject to additional attenuation. For a frequency of 64-128 MHz, this overlap corresponds to approximately 2-3X the maximum aperture dimension.

Therefore, depending upon the specific application and shield design, a minimum shield overlap dimension should be 2- 3X the maximum aperture dimension.

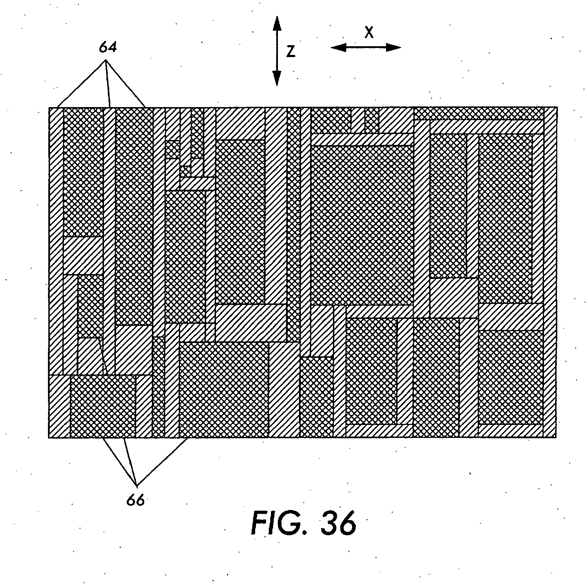

Figure 36 illustrated a top view of a fabricated layer of the shielding. As illustrated in Figure 36, the conductive material **64** is patterned or apertured in such a way that the widths of the non-conductive materials **66** in the z-direction, the z-direction representing the planar surface of a layer as it is wrapped around a lead, are random. More specifically, the conductive materials **64** are patterned or apertured in such a way that non-conductive materials **66** spaced in a random manner. Lastly, the spacing between non-conductive materials **66** in an x-direction, the x-direction being substantially parallel to an axis of a lead, is also random.

It is noted that the although Figures 32-36 show a patterned or apertured shielding with respect to a lead, the patterned or apertured shielding can be used to shield other shaped devices such as electrical component housings or other components of a medical assist device/ system.

It is further noted that the actual dimensions of the non-conductive materials can be set to suppress the radiation and eddy currents induced from specific frequencies; e.g., the lengths of the non-conductive material can be varied to suppress or block specific or undesired radiation frequencies. The dimensions of the non-conductive material would be on the order of the wavelength of the radiation so as to suppress, block, or shield the radiation.

Preferably, the distance of the overlap is much smaller than the wavelength of the electromagnetic pulse that the shield must attenuate to prevent the incident pulse from passing unattenuated through the shield.

The actual layers may be formed using a photoresist/masking process such as used in integrated circuit fabrication and thin film deposition. By using a mask/ photoresist process, the conductive/insulative layers in the vertical direction can be easily formed so that the conductive/insulative layers alternate (radially outward from the axis of the lead), and the conductive/insulative layers in the horizontal direction also alternate (substantially parallel to an axis of the lead).

Although, as stated above, eddy currents in a conductive structure may cause heating to the surrounding tissue, eddy currents induced in the tissue may also cause the heating of the surrounding tissue. As noted above, when a substance such as human tissue is subjected to a static magnetic field, the individual magnetic moments of the spins in the tissue align in a parallel and anti- parallel direction with the static magnetic field. This direction along the static magnetic field can be termed as the longitudinal direction. In magnetic- resonance imaging, the radio frequency polarizing field used for spin manipulation is constantly changing and thus, the individual magnetic moments of the spins in the tissue attempt to align with the polarizing field. The constant changing of alignment of the magnetic moments of the spins in the tissue causes the tissue's temperature to increase, thereby exposing the tissue to possible magnetic- resonance imaging induced thermal damage. Thus, this second possible cause of undesirable heat needs to also be addressed to prevent tissue damage caused by excessive heat.

The heat being accumulated in the surrounding tissue is addressed and reduced or prevented, notwithstanding the source or cause of the excessive heat. To better describe this the term, specific absorption ratio, will be used in discussing the excessive heat.

The specific absorption ratio is defined as the amount of power absorbed by a sample within a magnetic-resonance imaging scanner. The sample could be a suitable phantom mimicking tissue properties or a tissue region of patient.

As noted above, this excessive heat could be caused by either eddy currents induced in conductive structures or eddy currents induced in the actual tissue. It is noted that although two distinct sources of excessive heat are identified, the actual source of the heat may not be known, nor is it a real consideration in the actual functionality.

With respect to induced eddy currents, due to their electromagnetic receptive capabilities, cardiac assist device leads, metallic guidewires, neurostimulator leads and intraluminal coil leads may induce a localized increase in the specific absorption ratio, thereby resulting in an increased heating of the surrounding tissue. Moreover, due to the individual magnetic moments of the spins in the tissue attempting to align with the polarizing field, the acquisition sequence of the magnetic-resonance imaging process may also induce a localized increase in the specific absorption ratio, thereby resulting in an increased heating of the surrounding tissue. This tissue heating, notwithstanding the source, may be a function of the time of radio-frequency excitation, gradient coil switching speed, or strength of the static magnetic field.

The localized specific absorption ratio of tissue is reduced by altering the actual magnetic-resonance imaging acquisition process. More specifically, the localized specific absorption ratio of tissue is reduced by varying the imaging pulse sequence and/or changing the timing parameters of magnetic-resonance imaging acquisition process. For example, in response to increases in the localized specific absorption ratio or during magnetic-resonance image acquisition, the radio-frequency excitation cycle may be interrupted to allow for cooling of the tissue. In other words, if a threshold temperature level or a localized specific absorption ratio is exceeded, the magnetic-resonance imaging acquisition can be halted until the tissue temperature or localized specific absorption ratio decreases to an accepted level.

It is noted that the increased in the localized specific absorption ratio may or may not have resulted from placement of a conductive structure near the tissue region.

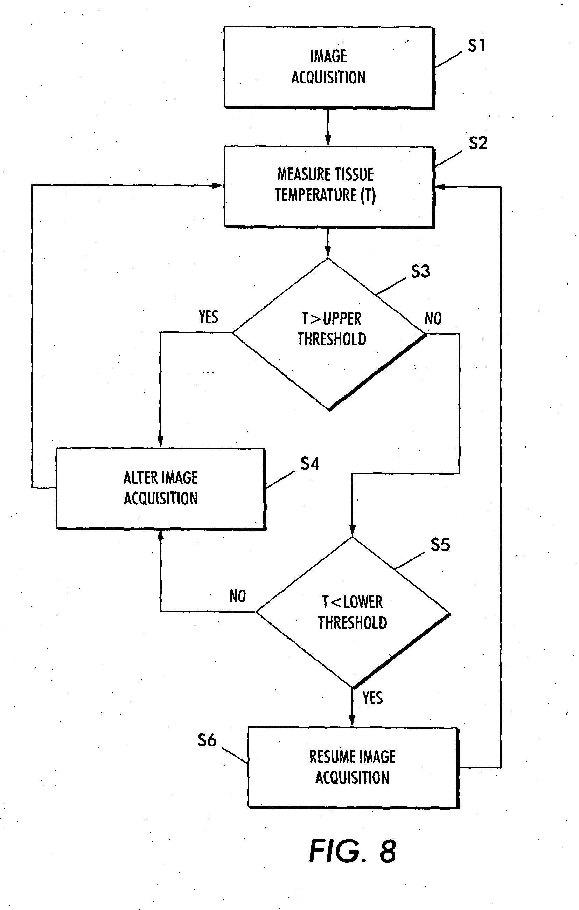

Figure 8 illustrates a magnetic-resonance imaging process according to the concepts of the present invention, which alters the actual magnetic-resonance imaging acquisition process in response to a temperature change in the tissue region being imaged. In this process, at Step S1, an image acquisition sequence is started by the magnetic-resonance imaging system. At step S2, the temperature change in the tissue region being imaged is measured. It is noted that this temperature change may be relative or absolute. The temperature change in the tissue region being imaged can be measured a number of ways.

For example, the relative temperature change can be monitored by magnetic image acquisition techniques. More specifically, relative temperature changes can be measured using a double gradient echo sequence to acquire a baseline phase image and a second phase image at within a single radio-frequency excitation of one same pulse sequence. A relative spatial temperature map of the desired region of interest can be calculated based on the subtraction of the two phase images collected during the double gradient echo sequence acquisition.

A more detail description of this relative temperature change acquisition process is described in US Patent Number 5,711,300, entitled "Real Time In Vivo Measurement Of Temperature Changes With Magnetic-resonance Imaging."

It is noted that this is only one example of collecting phase image data for relative temperature mapping. Other methods for collecting phase image data for relative temperature mapping may also be used.

It is further noted that absolute temperature changes can be determined using known methods, such as fiber optic thermocouples attached to guidewires or catheters, or non-contact thermographic imaging techniques.

Upon calculating the temperature change of the desired tissue region, such as described above with respect to the relative spatial temperature map of the desired region of interest, at Step S3, a predetermined upper temperature threshold is compared with the calculated temperature change, or in the example described above, compared with the relative temperature map. If the temperature change exceeds or is equal to the predetermined upper temperature threshold, the imaging acquisition sequence is adjusted or halted so as to reduce the temperature change below the predetermined upper temperature threshold, at Step **S4**.

The process returns to Step **S2** to make another determination of the temperature change in the tissue region of interest. If the temperature change is below the predetermined upper temperature threshold, Step **S5** determines if the measured temperature change is above a predetermined lower temperature threshold. If Step **S5** determines that the measured temperature change is above a predetermined lower temperature threshold, the process returns to Step **S4** and the imaging acquisition sequence remains adjusted or halted so as to reduce the temperature of the tissue region of interest. On the other hand, if Step **S5** determines that the measured temperature change is below or is equal to a predetermined lower temperature threshold, the process resume image acquisition at to Step **S6**.

It is to be understood that this type of adjustment can be applied to any form of pulse sequence or imaging acquisition method so as it meets the requirements of the user. There are an infinite number of variations in pulse sequence design and pulse sequence parameter determination to minimize heating. The actual variation in pulse sequence design and pulse sequence parameter determination will be dependent upon the image quality and the temporal resolution of acquisition desired; however, the required tissue temperature threshold(s) will be the ultimate determinant.

This may also include a user interactive process, which can be programmed. An example of such a process is described in detail in US Patent Number 6,396,266, entitled "magnetic-resonance Imaging System With Interactive magnetic-resonance Geometry Prescription Control."

In another arrangement, the SAR coefficients of a wide range of implantable medical devices are programmed into the magnetic-resonance imaging system, which then uses these data to predetermine and apply as safe magnetic-resonance imaging sequence.

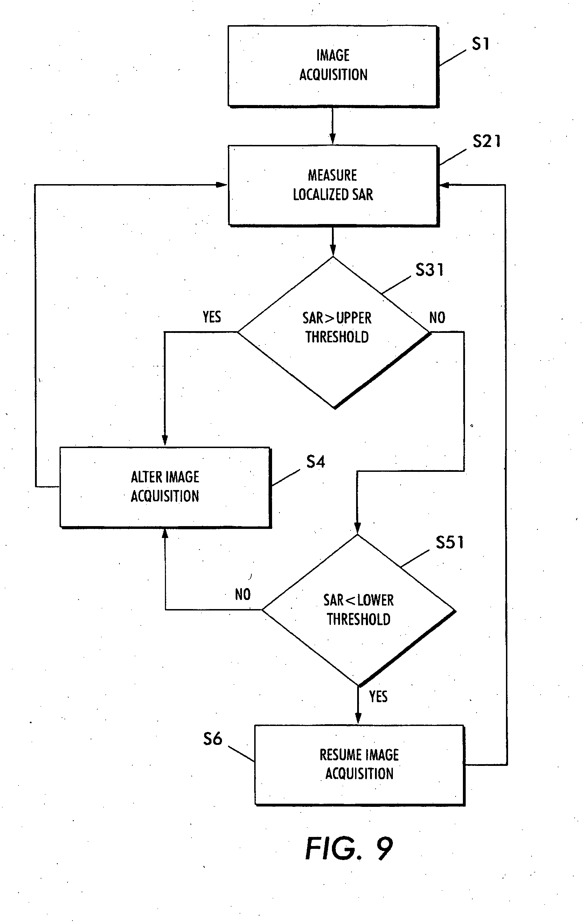

As illustrated in Figure 9, a magnetic-resonance imaging process, according to the concepts of the present invention, alters the actual magnetic-resonance imaging acquisition process in response to a calculated specific absorption ratio of the tissue region being imaged. In this process, at Step **S1**, an image acquisition sequence is started by the magnetic-resonance imaging system. At step **S21,** the specific absorption ratio is estimated using theoretical modeling and/or empirical results from certain conductors being imaged in tissues. At Step **S31,** the calculated specific absorption ratio level (s) are then be compared to threshold levels.

If the calculated specific absorption ratio level exceeds or is equal to the predetermined upper specific absorption ratio level threshold, the imaging acquisition sequence is adjusted or halted so as to reduce the temperature of the tissue region of interest, at Step **S4.**

The process returns to Step **S21** to make another determination of the specific absorption ratio level. If the determined specific absorption ratio level is below the predetermined upper specific absorption ratio level threshold, Step **S51** determines if the determined specific absorption ratio level is above a predetermined lower specific absorption ratio threshold. If Step **S51** determines that the determined specific absorption ratio level is above a predetermined lower specific absorption ratio threshold, the process returns to Step **S4** and the imaging acquisition sequence remains adjusted or halted so as to reduce the temperature of the tissue region of interest. On the other hand, if Step **S51** determines that the determined specific absorption ratio level is below or is equal to a predetermined lower specific absorption ratio threshold, the process resume image acquisition at to Step **S6**.

This may also include a user interactive process described above.

As illustrated in Figures 8 and 9, specific absorption ratio or energy deposition of radio frequency energy in the tissue can be reduced by specific manipulations of each individual radio frequency pulse. Moreover, these embodiments of the present invention may manipulate the pulse waveform, pulse duration and pulse amplitude to reduce the specific absorption ratio. The choice of the integrated values of these three parameters is determined upon by the requirements of the user. For example, reduction of the amplitude and increase in the duration of the pulse may reduce the specific absorption ratio, but this may not be appropriate for the specified timing required to image certain contrast parameters.

A more detail discussion of the specific manipulations that be used to reduce specific absorption ratio is set forth in US Patent Number 4,760,336, entitled "Variable Rate Magnetic Resonance Selective Excitation For Reducing radio-frequency Power And Specific Absorption Rate."

It is also noted that Figures 8 and 9 can be used not only as a stand-alone solution to reduce the increases in specific absorption ratio resulting from conductive structures in tissue, but also in conjunction with the patterned shielding embodiments described above.

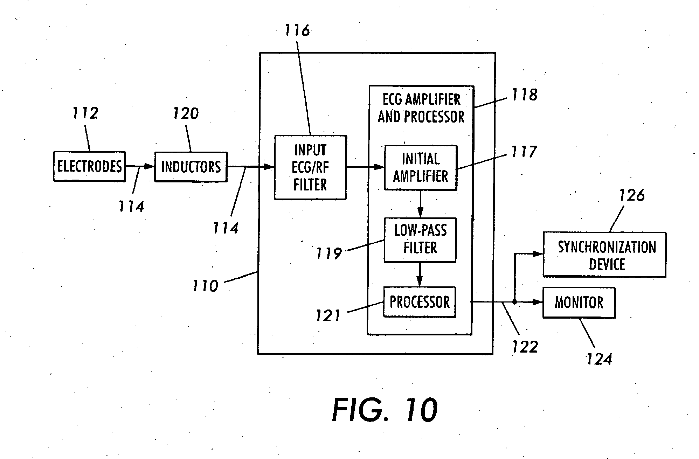

A block diagram of an ECG acquisition and processing system is shown in Figure 10. The ECG monitoring system can be used as a stand-alone ECG monitor or as part of an external or internal pacemaker. A patient is connected to ECG monitoring circuitry **110** via surface or internal electrodes **112** and lead wires **114**. The lead wires **114** connect to the input ECG/radio-frequency filter **116**.

This filter design enables the monitoring circuit to operate in the high radio-frequency field environment generated by the magnetic-resonance imaging system. First, the radio-frequency filter prevents the development of excess voltage on the ECG input amplifier and processor **118** that would otherwise damage the amplifier, and excess voltage on the ECG leads potentially inducing a dangerous heart rhythm. Second, it prevents the development of excess current on the ECG electrodes **112** that would induce thermogenic damage at the electrode-heart tissue interface that could lead to loss of capture of pacing signals and possibly death. Third, the radio-frequency filter **116**, when attached between the lead wires **114** and the ECG input amplifier, will not interfere with proper sensing of the ECG signal.

The input ECG/radio-frequency filter **116** has the characteristics of a low-pass filter with approximately 100 dB signal attenuation in the commonly used magnetic-resonance imaging system radio-frequency frequency range. The frequencies contained in the intracardiac or surface ECG signal, however, are passed without any attenuation.

In the case where the lead wires **114** are of long length, such as in external monitoring, there is significant stray capacitance between the lead wires. As a result, electromagnetic fields generated between the electrodes **112** can produce dangerously high voltage within and current flow through the electrodes.

To limit this voltage and current, inductors **120**, may be placed in the lead wires **114**, close to the electrodes. The ECG/radio-frequency input filter **116** and lead wire inductors **120** must contain components with a resonance frequency higher than the radio-frequency frequency of the magnetic-resonance imaging system in use.

The ECG amplifier/processor **118** contains low-pass or band reject filter **119** placed after the initial amplifier **117** and before final stages **121**. The low-pass or band reject filter must pass the QRS signals and reject gradient field and radio-frequency frequencies. The output ECG signal **122**, which can take any conventional form such as an R-wave detector output or analog ECG, can be used for any purpose. It can be used to display the ECG on monitor **124**, or synchronize an external or internal device **126**, such as a magnetic-resonance imaging system or cardiac pacemaker.

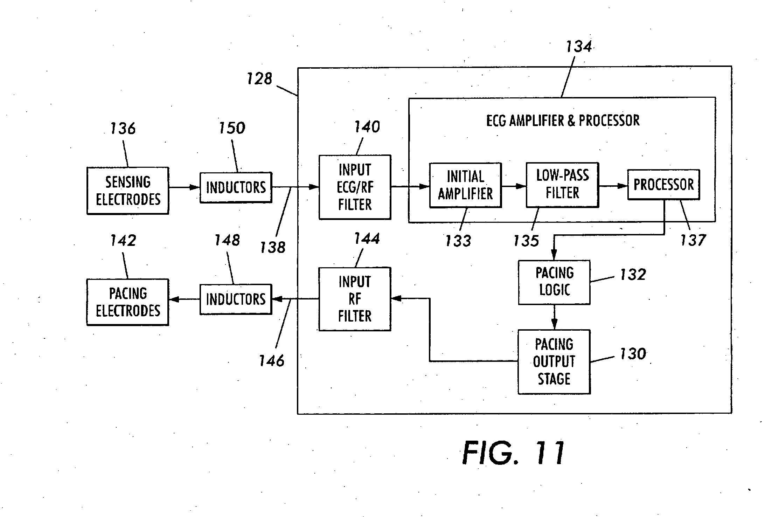

As shown in Figure 11, a pacemaker system **128** is described This pacemaker system can be either external or implantable. In addition to the elements shown in Figure 10, this also contains a pacing output stage **130** that is controlled by the pacing logic **132**.

The pacing logic is itself controlled by signals from the ECG amplifier/processor **134**. The patient is connected to the pacemaker system **128**, via surface or internal sensing electrodes **136** and lead wires **138**. The lead wires are connected to the ECG amplifier/processor **134**, via the input ECG/radio-frequency filter **140**. The pacing output stage **130** is connected to pacing electrodes **142** via the output radio-frequency filter **144** and pacing lead wires **146**. The ECG amplifier/processor **134** contains a low-pass or band reject filter **135** placed after the initial amplifier **133** and before the final stages **137**.

The low-pass or band reject filter must pass the QRS signal and reject gradient field frequencies caused by the magnetic-resonance imaging. As in the previous embodiment, inductors **148, 150** may be placed in the lead wires **138, 146** close to the electrodes **136, 142. The** sensing **136** and pacing **142** electrodes, wires **138, 146,** inductors **148, 150,** and radio-frequency filters **149, 144** are shown separately. However, these components can be merged to create a combined ECG sensing/pacing radio-frequency electrodes, wires, inductors, and filters.

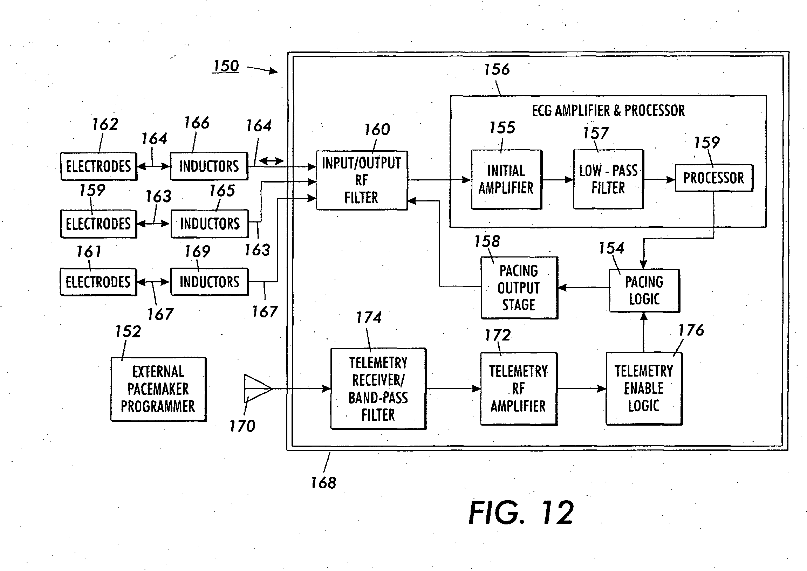

An implantable pacemaker **150** capable of bi-directional communication with an external programmer **152** is shown in Figure 12. The implantable unit contains conventional programmable pacing logic **154** for sensing and/or pacing the ventricle and/or atrium and includes an ECG amplifier and processor **156,** pacing output stage **158,** combined input/output radio-frequency filter **160**, electrodes **162** and leads **164**. The ECG amplifier and processor **156** contains a low-pass or band reject filter **157** placed after the initial amplifier **155** and before final stages **159**. The low-pass or band reject filter must pass the QRS signals and reject gradient field frequencies caused by the magnetic-resonance imaging.

inductors **166** may be placed in the lead wires **164** close to the electrodes **162**. The electrodes **162**, leads **164**, inductors **166**, and input/output filter **160** may be single elements performing combined input and output functions or may be separate input and output elements. For dual chamber pacemakers, multiple sets of sensing/pacing electrodes may be used **159, 162,** which are each connected to input/output radio-frequency filters **160**.

A first electrode pair may be for atrium sensing or pacing and a second electrode pair may be for ventricle sensing or pacing. As is taught in the pacing art, pacing logic **154** may be programmed, for example, to sense the atrium signal and pace via the ventricle electrodes. In addition, a new form of circulatory support called cardiomyoplasty may utilize other electrodes **161** and leads **167** to pace skeletal muscle wrapped around the heart and stimulated in synchrony with pacing the heart. In each type of pacemaker the leads **163**, **164**, **167** may contain inductors **165**, **166**, **169** to reduce electrical currents across the electrodes and each lead may be connected to separate input/output radio-frequency filter **160**.

In order to protect the pacemaker from the effects of the radio-frequency field generated by the magnetic-resonance imaging system, it is necessary to surround the components with a radio-frequency shield **168**. In a preferred embodiment of the present invention, this shield is as described above with respect to Figures 3 through 7.

More specifically, the shield may consist of patterned layers including conductive materials and non-conductive materials. The conductive materials of the present invention may be a metal, a carbon composite, nanotubes (wherein the nanotubes may be constructed from a carbon base or the nanotubes could be formed from other amalgams coated with the appropriate material (s) ) , metal- coated carbon filaments (wherein the metal may be one of the following metals: nickel, copper, cobalt, silver, gold, tin, or zinc) , metal- coated ceramic filaments (wherein the metal may be one of the following metals: nickel, copper, cobalt, silver, gold, tin, or zinc) , a composite of metal- coated carbon filaments and a polymer (wherein the polymer may be one of the following: polyether sulfone, silicone, polymide, polyvinylidene fluoride, or epoxy) , a composite of metal- coated ceramic filaments and a polymer (wherein the polymer may be one of the following: polyether sulfone, silicone, polymide, polyvinylidene fluoride, or epoxy) , a composite of metal- coated carbon filaments and a ceramic (wherein the ceramic may be one of the following: cement, silicates, phosphates, silicon carbide, silicon nitride, aluminum nitride, or titanium diboride) , a composite of metal-coated ceramic filaments and a ceramic (wherein the ceramic may be one of the following: cement, silicates, phosphates, silicon carbide, silicon nitride, aluminum nitride, or titanium diboride) , or a composite of metal- coated (carbon or ceramic) filaments (wherein the metal may be one of the following metals: nickel, copper, cobalt, silver, gold, tin, or zinc) and a polymer/ ceramic combination (wherein the polymer may be one of the following: polyether sulfone, silicone, polymide, polyvinylidene fluoride, or epoxy and the ceramic may be one of the following: cement, silicates, phosphates, silicon carbide, silicon nitride, aluminum nitride, or titanium diboride) .

Since the implantable pacemaker shown in Figure 12 is enclosed in a radio-frequency shield, it was necessary to develop a unique method of external programming. The information received by the pacemaker **150** is processed to separate the desired communication signal from the radio-frequency signals produced by the magnetic-resonance imaging system. An antenna **170** is connected to the telemetry radio-frequency amplifier **172** via the telemetry receiver/band pass filter **174**. This filter is a band pass filter that passes only the specific programming frequency to the telemetry amplifier **172**.

The telemetry logic circuit **176** interprets all radio-frequency received by the telemetry antenna **170** and will only allow programming of the pacing logic circuit **154** when a specific telemetry enable pattern is received. The telemetry enable logic circuit **176** will also inhibit control of the pacing output stage **158** while programming is taking place. This prevents improper and potentially dangerous pacing parameters from controlling the pacing stage. As a further safety measure, the telemetry enable circuit 176 enables pacing at a preset rate ("safety pacing") to provide adequate pacing back-up for pacemaker dependent patients during external programming.

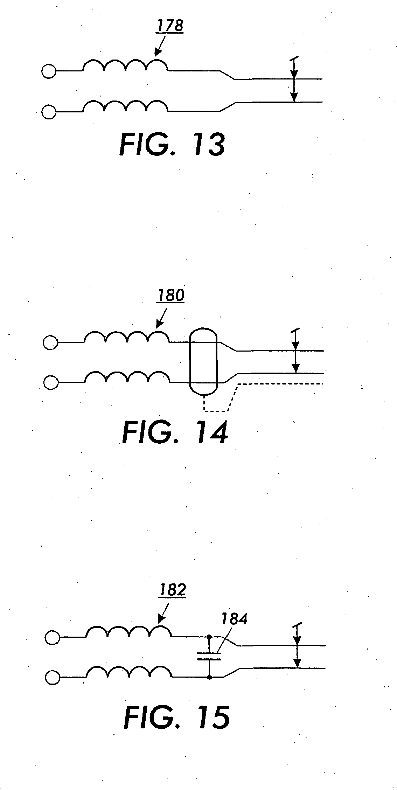

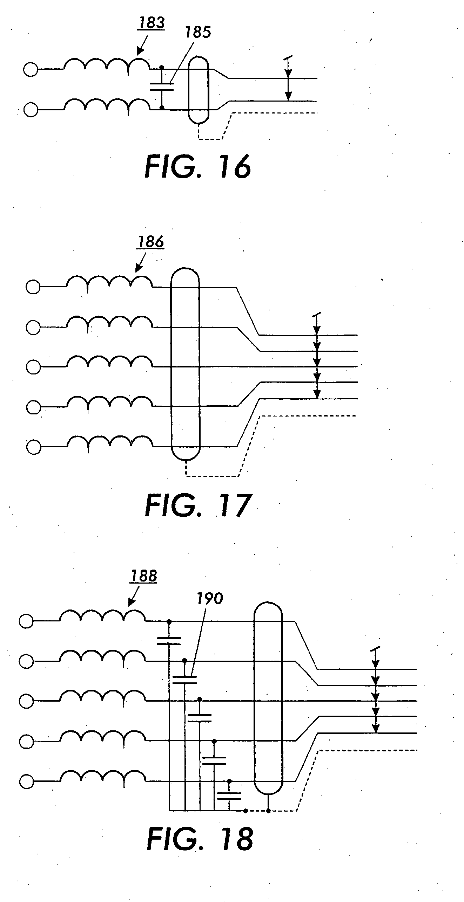

Figures 13 through 18 are schematic diagrams of the inductor elements (elements **120**, **148**, **150**, **165**, **166**, and **169**) that are placed in the lead wires to reduce harmful currents. As mentioned previously, significant stray capacitance can be produced along the lead wires, particularly for external pacers. As a result, electromagnetic fields generated between the electrodes because of the radio-frequency field can produce dangerously high voltages across and currents flowing through the electrodes. To limit this current, and enhance patient safety, the inductor elements shown in Figures 13 through 18 are placed in the lead wires close to the electrodes. Figure 13 shows the inductor elements **178** when a two-wire lead is used. Figure 14 shows the inductor elements **180** when a two-wire shielded lead is used. Figure 15 shows alternative inductor elements **182**, which incorporate a capacitor **184**, thus comprising an additional low-pass L-C filter. Figure 16 shows the same alternative inductor elements **183** and a capacitor **185**, as shown in Figure 15, but using a shielded two-wire lead. Figure 17 shows the inductor elements **186** used in a multi-lead shielded harness. Figure 18 shows alternative inductor elements **188** used in a multi-lead shielded harness, which also includes capacitor components **190** connected to the shielding enclosure of the capacitors.

The leads can be used to measure ECG, EEG, or other electrical signals of physiological significance. Figures 13 through 18 show twisted leads, but it is to be understood that coaxial as well as other forms of lead wires could be used.

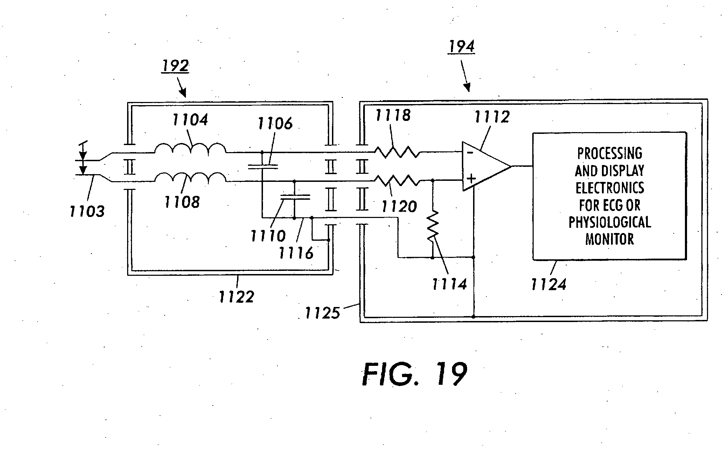

Figures 19 through 31 show the input radio-frequency filter, output radio-frequency filter, and combined input/output radio-frequency filter. According to the concepts of the present invention, there are many possible specific implementations of the radio-frequency filter design that will depend on: 1) whether two leads or multiple leads are used; 2) whether the leads are shielded; 3) whether single or multiple stage filtering is used; and 4) whether each radio-frequency filter is housed in a separate shielded enclosure.

It should be apparent that the filters could be placed either at the proximal end or the distal end of the pulse generator - lead assembly.

It will also become apparent that the same filter design principles can be used whether the filter is acting as an input filter for an ECG monitor, as separate input filters and output filters for a pacemaker, or as a single combined input/output filter for a pacemaker.

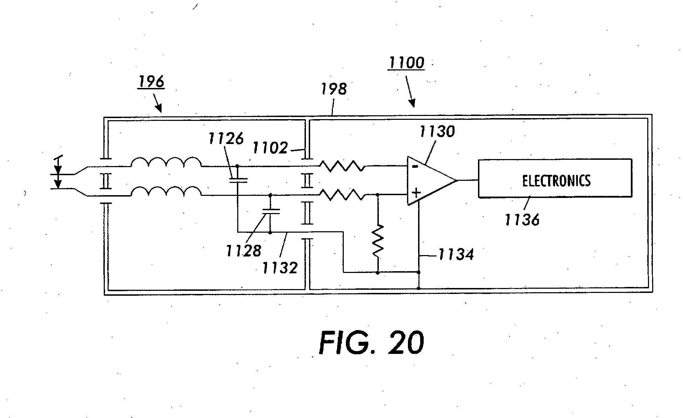

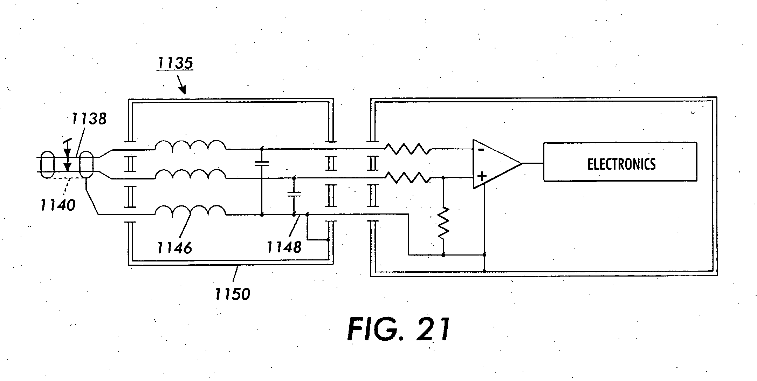

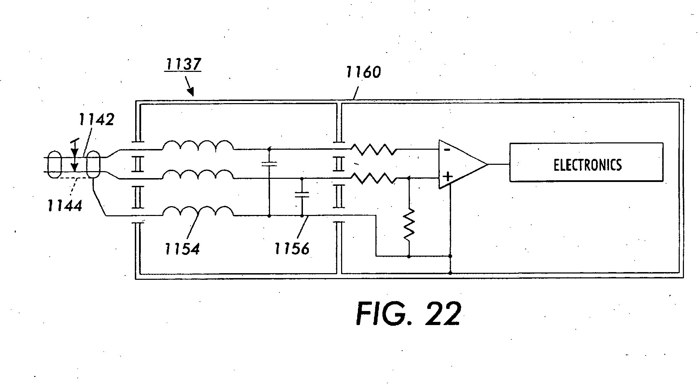

Figures 19 and 20 show one-stage filters for use with two unshielded leads. Figures 21 and 22 show alternative ways to shield the electronics.

Figure 19 shows the input filter **192** in a separate shielded enclosure; whereas Figure 20 shows the input filter **196** located in the same-shielded enclosure **198** as the processing electronics **1100** with an internal wall **1102** shielding the monitoring electronics **1100** from the input filter **196**. (Whether one or two enclosures are used, the filter section should be shielded from the other circuitry.)

As can be seen in Figure 19, each lead is connected to a separate low-pass filter. (Leads **1103** may be twisted to reduce electromagnetic interference). A first low-pass filter is made from inductor **1104** and capacitor **1106**; and a second low-pass filter is made from inductor **1108** and capacitor **1110**. Each low-pass filter offers approximately 100 dB signal attenuation in the radio-frequency frequency range of the magnetic-resonance imaging system and passes with little attenuation the desired physiological signal.

As mentioned previously, the L-C components must be high frequency elements retaining their desired characteristics throughout the frequency range. The output from the input filter **192** is connected to differential amplifier **1112**. In order to properly utilize the differential amplifier **1112**, the non-inverting input of the ECG amplifier **1112** is connected to the amplifier zero-signal reference terminal via a resistor **1114**, as is well known in the art.

Obviously, any other method of converting a two to three conductor input may also be used. The two capacitors **1106**, **1110** in the low- pass filters are referenced to a third wire **1116** that is connected to the shield surrounding the filter **1122** and also connected to the zero-signal reference terminal of the ECG amplifier **1112**.

The ECG amplifier may be a single differential amplifier or a combination of multiple differential amplifiers. Alternatively, capacitors **1106**, **1110** can be connected directly to the shield **1122** with a separate wire connecting the shield **1122** to the zero-signal reference terminal of the ECG amplifier **1112**.

To improve the characteristics of the filter, resistors **1118**, **1120** may be added between the input filter **192** and the input of the differential amplifier **1112**. Just as the input filter **192** is enclosed in a shield **1122**, the ECG amplifier **1112** and processor **1124** may also be enclosed in a separate shield **1125**.

The shields may consist of patterned layers including conductive materials and non- conductive materials. The conductive materials of the present invention may be a metal, a carbon composite, nanotubes (wherein the nanotubes may be constructed from a carbon base or the nanotubes could be formed from other amalgams coated with the appropriate material (s) ) , metal- coated carbon filaments (wherein the metal may be one of the following metals: nickel, copper, cobalt, silver, gold, tin, or zinc) , metal- coated ceramic filaments (wherein the metal may be one of the following metals: nickel, copper, cobalt, silver, gold, tin, or zinc) , a composite of metal-coated carbon filaments and a polymer (wherein the polymer may be one of the following: polyether sulfone, silicone, polymide, polyvinylidene fluoride, or epoxy) , a composite of metal- coated ceramic filaments and a polymer (wherein the polymer may be one of the following: polyether sulfone, silicone, polymide, polyvinylidene fluoride, or epoxy) , a composite of metal- coated carbon filaments and a ceramic (wherein the ceramic may be one of the following: cement, silicates, phosphates, silicon carbide, silicon nitride, aluminum nitride, or titanium diboride) , a composite of metal- coated ceramic filaments and a ceramic (wherein the ceramic may be one of the following: cement, silicates, phosphates, silicon carbide, silicon nitride, aluminum nitride, or titanium diboride) , or a composite of metal- coated (carbon or ceramic) filaments (wherein the metal may be one of the following metals: nickel, copper, cobalt, silver, gold, tin, or zinc) and a polymer/ ceramic combination (wherein the polymer may be one of the following: polyether sulfone, silicone, polymide, polyvinylidene fluoride, or epoxy and the ceramic may be one of the following: cement, silicates, phosphates, silicon carbide, silicon nitride, aluminum nitride, or titanium diboride) .

Figure 20 shows the input filter **196** and processing electronics **1100** are housed in a single contiguous shielding enclosure **198**. A separate metallic wall **1102** is used to shield the electronics **1100** from the input filter **196**. The two capacitors **1126**, **1128** that make up the low-pass filters are connected to the zero-signal reference terminal of the differential amplifiers **1130** via a third reference wire **1132**; the reference wire **1132** and zero-signal reference terminal **1134** both connected to the pacemaker shielding case at a single point.

Both Figures 19 and 20 show processing electronics **1224**, **1136** that are used to further process the signal obtained from the differential amplifiers **1112**, **1130**. The processing electronics may be used to process and display the ECG signal, in the case of an external monitor, or be used to process and control pacing in the case of a pacemaker. It is to be understood that the filter arrangements shown in Figures 19 and 20 could be used in a device that monitors physiologically significant electrical signals other than ECG.

Figures 21 and 22 show one-stage filter designs **1135** and **1137** that is preferred when the input leads are shielded. The design is identical to that shown in Figures 19 and 20, except that the lead shield must be coupled to the shield enclosure via a radio-frequency filter. To accomplish this, the lead shield **1140** of the leads **1138**, in Figure 21, is connected by an inductor **1146** to the third reference wire **1148**, and the reference wire is connected to the filter's shielding enclosure **1150**. Analogously, the shield **1144** of the leads **1142** in Figure 22 is connected by an inductor **1154** to the reference wire 1156, connected in turn to the enclosure 1160. In order to further reduce the effects of the magnetic-resonance imaging system radio-frequency signal, more than one stage of filtering can be used.

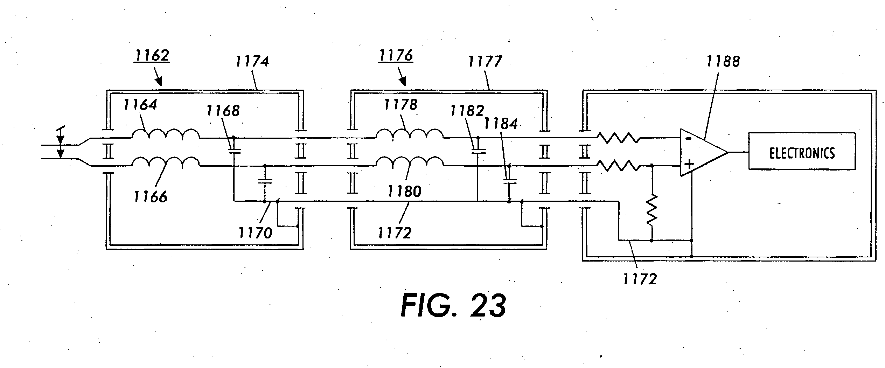

As shown in Figure 23, two stages of filtering are used. Each filter stage is similar to the filter described in Figure 19. Each input lead is connected to a low-pass filter. Each low-pass L-C filter has a capacitor connected to a reference wire that is connected to the shielding enclosure.

The first stage filter **1162** contains low-pass filters comprising inductors **1164**, **1166** and capacitors **1168**, **1170**. Capacitors **1168**, **1170** are coupled to a reference wire **1172** that is connected to the enclosure **1174**. Similarly, the second filter stage **1176** contains low-pass filters made from inductors **1178**, **1180** and capacitors **1182**, **1184**, with capacitors **1182**, **1184** coupled to the reference wire **1172** that is connected to the shielding enclosure **1177**. All the capacitors (**1168**, **1170**, **1182** and **1184**) used in the low-pass filters are connected to the reference wire **1172** which is connected to the zero-signal reference terminal of the differential amplifier **1188**.

Each stage in Figure 23 is housed in a separate shielding enclosure and each shielding enclosure is coupled to the reference wire **1172**. It is understood that this concept can be extended to multistage filtering. The second stage **1176** could actually represent the Nth stage of a multistage filter, with each identical stage coupled together as shown.

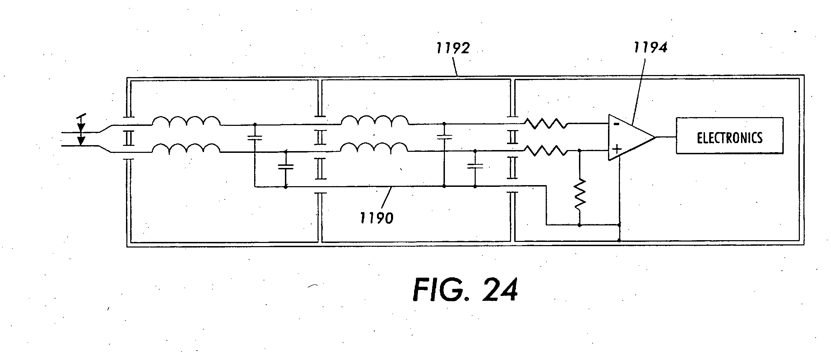

A multiple stage filtering is shown in Figure 24. Each filtering stage as well as the differential amplifier and the associated electronics are housed in the same contiguous shielding enclosure **1192**. The filter design in this arrangement is identical to that shown in Figure 20; however, the reference wire is only connected to the shielding case **1192** at a single point. In effect, the low-pass filters in each stage and the zero-signal reference terminal of the differential amplifier **1194** are all referred to the same point on the shielding enclosure. It is to be understood that Figure 24 may also have three or more stages, each stage shielded and coupled together as shown.

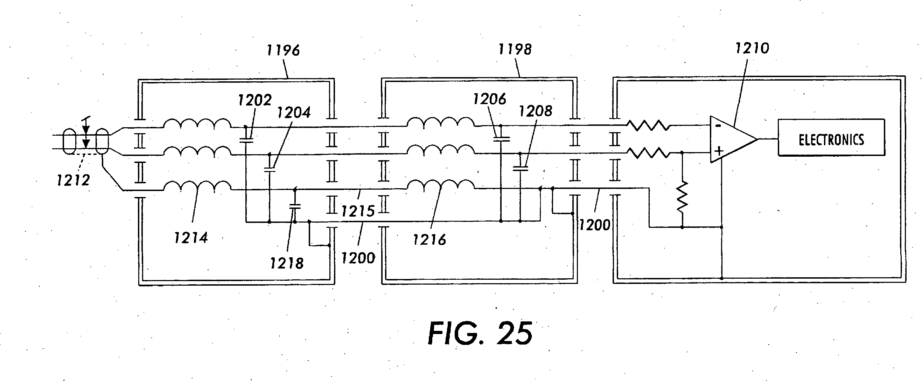

Figures 25 and 25 are schematic drawings of a two-stage filter for use with shielded leads. These filters are similar to the multistage filter shown in Figures 23 and 24, except that the lead shields are coupled via inductors to the reference wire in the last filter stage. Figure 25 is a two-stage filter where each stage is separately isolated in enclosures **1196**, **1198**. Again, as in the previous embodiments, low-pass filters are connected in each input lead and referenced to a common reference line **1200** (i.e., capacitors **1202**, **1204**, **1206**, and **1208** are connected to the reference line **1200**). The common reference line **1200** is connected to the shielding enclosure for each stage and is input to the zero-signal reference terminal of the differential amplifier **1210**.

The lead shield 121**2** is connected to inductor **1214** in the first filter stage and via line **1215** to inductor **1216** in the second filter stage. The inductor **1216** in the second, or final filter stage, is connected to the common reference line **1290**. As discussed previously, the inductors **1214**, **1216** provide high impedance to the undesirable high frequency radio-frequency voltages produced by the magnetic-resonance imaging system and prevent introduction of radio-frequency interference into the processing electronics via the common wire.

To increase performance, an additional capacitor **1218** may be connected between lines **1215** and **1200** in the first stage and any subsequent filter stage other than the last stage. Inductor **1214**, coupled with capacitor **1218**, provides a third low-pass filter.

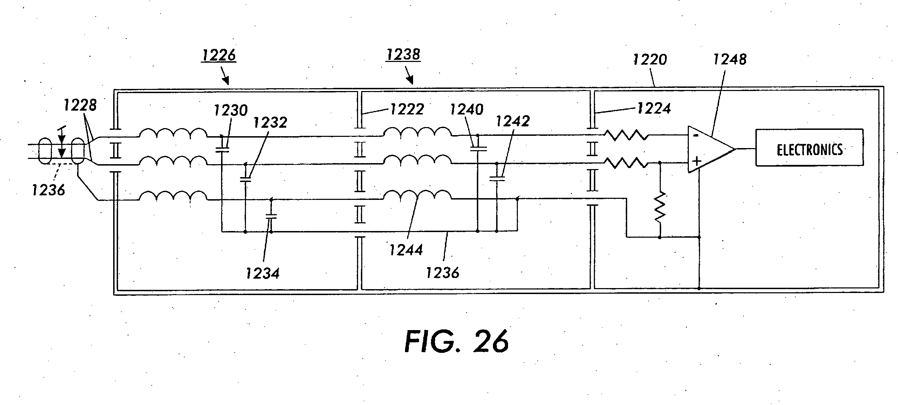

Figure 26 is a multistage filter housed in a single shielding enclosure **1220** with separate walls **1222**, **1224** isolating the filtering stages from each other and from the processing electronics. In the first stage **1226**, each lead **1228** and the lead shield **1230** are coupled to low-pass filters referenced by capacitors **1230**, **1232**, and **1234** to a common reference line **1236**. In the last stage **1238**, only the two lead wires contain low-pass filters referenced by capacitors **1240**, **1242** to the common reference line **1236**. The lead shield **1230** is coupled via inductor **1244** directly to the reference line **1236**.

As in the other arrangements, the reference line **1236** is connected to the zero-signal reference terminal of the differential amplifier **1248**. Also as in the other arrangements, when a single case is used, the common reference line **1236** is connected to the shielding enclosure at a single point. It is of course understood that the two-stage filters shown in Figures 25 and 26 could be easily extended to multiple stage filtering with each stage connected as described.

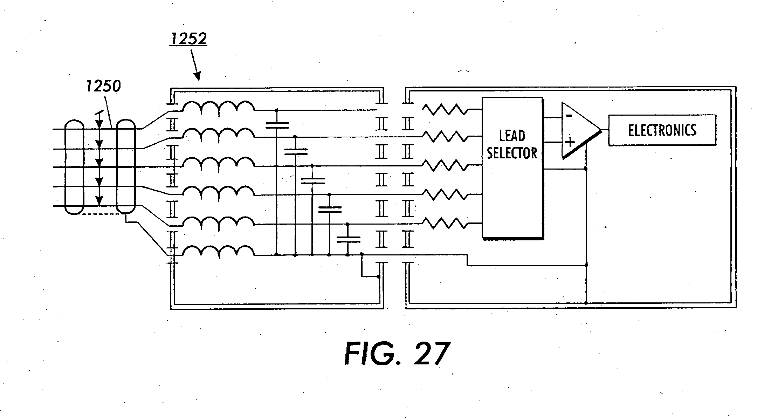

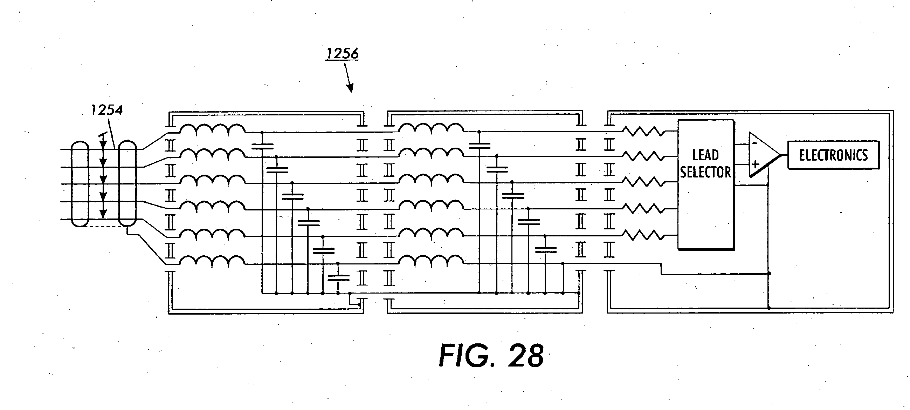

Figures 27 and 28 show an extension of the single and multiple stage filtering to multiple lead shielded ECG harness. In Figure 27, a five conductor shielded cable **1250** is filtered by a single stage filter **1252**. In Figure 28, a five conductor shielded cable **1254** is filtered by a multiple stage filter **1256**.

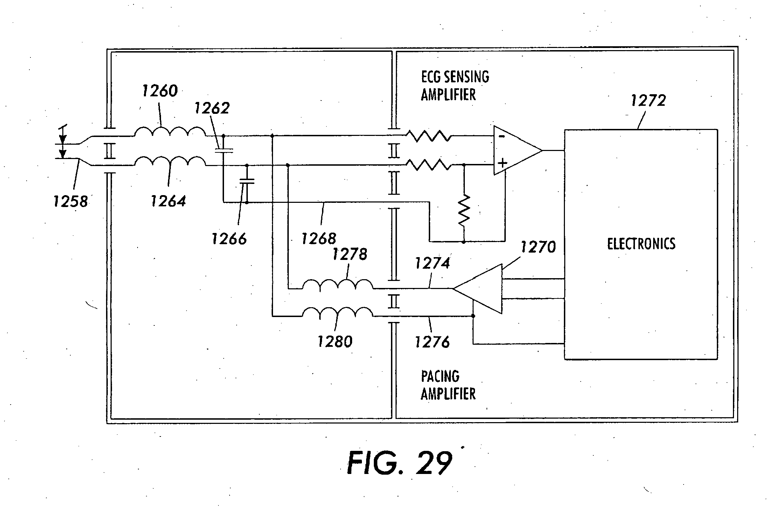

Thus far, the filters have been described as input filters. However, as shown in Figure 29, the filters can operate as both input and output filters. As generally true in pacemakers, the lead pair **1258** can be used to sense electrical activity of the heart as well as to pace the heart. As shown previously, low pass filters (i.e., inductor **1260**/capacitor **1262** pair and inductor **1264**/capacitor **1266** pair) for each lead are referenced to a common reference line **1268**. The reference line 1268 then connects to the zero-signal reference terminal of the differential amplifier and to the shielding enclosure. A pacing amplifier **1270** in controlled by electronics **1272** to generate a pacing signal. The pacing signal is connected via lines **1274**, **1276** through the filter, to the leads **1258**. Two inductors **1278**, **1280** are placed in lines **1274**, **1276** to further prevent any current generated by the magnetic-resonance imaging system from affecting the pacing amplifier **1270**; the inductors **1278**, 1 provide an additional high impedance to the high frequency radio-frequency signals produced by the magnetic-resonance imaging system.

Connecting the pacing amplifier to the leads **1258** via the filter can be used with each of the filter embodiments described above. In multistage filtering embodiments, pacing amplifiers would be preferably connected into the last filtering stage.

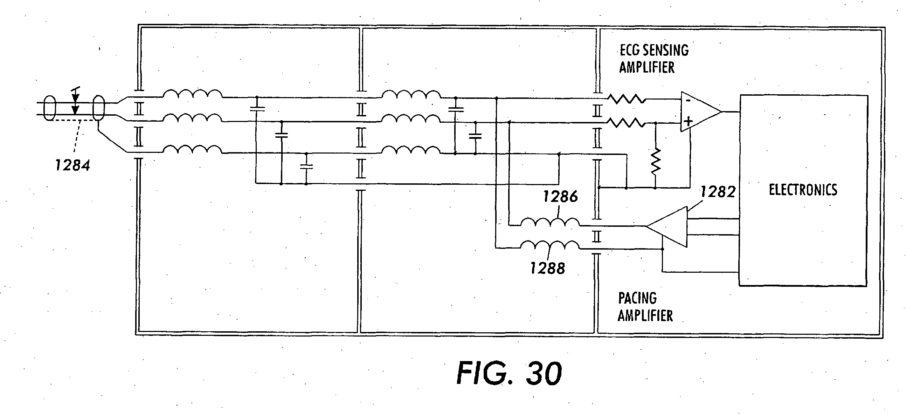

Figure 30 is an exemplary embodiment, showing how the pacing amplifier **1282** can be connected via a multi-stage filter to the shielded pacing/sensing leads **1234**. The pacing amplifier **1282** is connected via inductors **1286** and **1288** to the lead wires in the last stage of the filter. As discussed above, the inductors **1286**, **1288** provide additional protection from the high frequency radio-frequency signals generated by the magnetic-resonance imaging system.

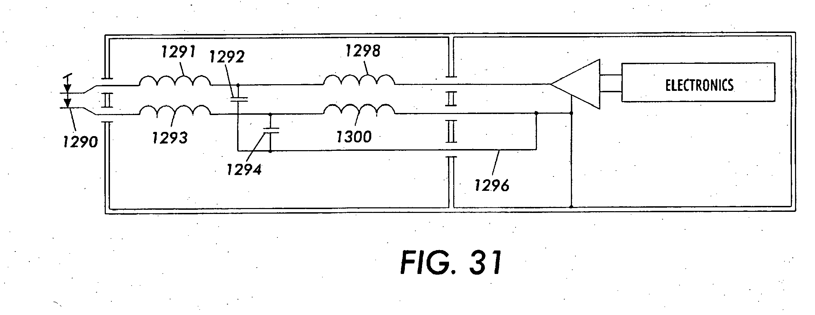

There are an unlimited number of ways in which the filters described herein can be connected to both sensing electronic and pacing amplifiers. In fact, the same filter design can be used in stimulators, as shown in Figure 31, where only a pacing signal is produced.

The filter design is identical to those described previously. The leads **1290** are each connected to low pass filters (inductor **1291**/capacitor **1292** pair and inductor **1293**/capacitor **1294** pair) that are referred by capacitors **1292**, **1294** to a common reference line **1296**. Inductors **1298**, **1300** provide additional isolation to protect the pacing amplifier. The filter design for stimulation use only can be extended to multistage embodiments as described above.

In order to protect the implantable pacemaker elements from the effects of the radio-frequency signals produced by the magnetic-resonance imaging system, it is necessary to surround the components with a radio-frequency shield.

This shielding may consist of patterned layers including conductive materials and non- conductive materials. The conductive materials of the present invention may be a metal, a carbon composite, nanotubes (wherein the nanotubes may be constructed from a carbon base or the nanotubes could be formed from other amalgams coated with the appropriate material (s) ) , metal- coated carbon filaments (wherein the metal may be one of the following metals: nickel, copper, cobalt, silver, gold, tin, or zinc) , metal- coated ceramic filaments (wherein the metal may be one of the following metals: nickel, copper, cobalt, silver, gold, tin, or zinc) , a composite of metal-coated carbon filaments and a polymer (wherein the polymer may be one of the following: polyether sulfone, silicone, polymide, polyvinylidene fluoride, or epoxy) , a composite of metal- coated ceramic filaments and a polymer (wherein the polymer may be one of the following: polyether sulfone, silicone, polymide, polyvinylidene fluoride, or epoxy) , a composite of metal- coated carbon filaments and a ceramic (wherein the ceramic may be one of the following: cement, silicates, phosphates, silicon carbide, silicon nitride, aluminum nitride, or titanium diboride) , a composite of metal- coated ceramic filaments and a ceramic (wherein the ceramic may be one of the following: cement, silicates, phosphates, silicon carbide, silicon nitride, aluminum nitride, or titanium diboride) , or a composite of metal- coated (carbon or ceramic) filaments (wherein the metal may be one of the following metals: nickel, copper, cobalt, silver, gold, tin, or zinc) and a polymer/ ceramic combination (wherein the polymer may be one of the following: polyether sulfone, silicone, polymide, polyvinylidene fluoride, or epoxy and the ceramic may be one of the following: cement, silicates, phosphates, silicon carbide, silicon nitride, aluminum nitride, or titanium diboride) .

In the various embodiments described above with respect to Figures 10 through 31, a common reference line was described in the context of a unipolar lead system. It is noted that the common reference line may not be a separate line, but the common reference line may be one of the leads in a bipolar lead system. Moreover, the common reference line may a type of ground path (or line) or signal return path (or line). The common reference line merely provides electrical ground for the filters.

In summary the present invention, as described above, is an electromagnetic shield having a first patterned layer having non-conductive materials and conductive materials and a second patterned layer having non-conductive materials and conductive materials. The conductive materials in the first patterned layer may be randomly located or located in a predetermined segmented pattern. Moreover, the conductive materials in the first patterned layer may be located in a predetermined segmented pattern with respect to locations of the conductive materials in the second patterned layer. The electromagnetic shield may further include a non-conductive layer between the first and second patterned layers such that the conductive materials in the first patterned layer can overlap the conductive materials in the second patterned layer with respect to a direction substantially perpendicular to a planar surface of the second patterned layer.

Therefore, the present invention provides a patterned shielding that substantially prevents undesired radiation from entering the lead from the radial direction or electrical component and also substantially prevents the formation or inducement of eddy currents on the surface of the shielding, which are induced by the pulsing of the radiation or changing magnetic field of the radiation. More specifically, the conductive material in a shielding layers of the present invention is patterned or segmented so that the conductive material is literally broken up to limit the build up of eddy currents induced by the changing magnetic fields.

Moreover, the present invention can reduce specific absorption ratio by altering the magnetic- resonance imaging acquisition process by altering the imaging pulse sequence and/or changing the timing parameters. Furthermore, the radio- frequency excitation cycle may be interrupted to allow for cooling. Therefore if a threshold temperature level is exceeded, the imaging acquisition can be halted until the tissue temperature is at an acceptable level.

While various examples and embodiments of the present invention have been shown and described, it will be appreciated by those skilled in the art that the scope of the present invention are not limited to the specific description and drawings herein, but extend to various modifications and changes all as set forth in the following claims.

**Claims**

1. A lead system, comprising:

a lead (60) to provide an electrical path to a tissue region; and
a shielding formed around said lead to shield said lead from electromagnetic interference; **characterised in that** :

said shield being formed of a first layer (80) patterned with non-conductive materials (66) and conductive material (64), and a second layer (82) patterned with non-conductive materials and conductive materials; wherein the conductive materials (64) of the first layer (80) are patterned in a direction substantially parallel to an axis of the lead system such that each of the conductive materials (64) is physically separated from the other conductive materials (64) of the first layer (80), wherein the conductive materials (64) of the second layer (82) are patterned in a direction substantially parallel to an axis of the lead system such that each of the conductive materials (64) is physically separated from the other conductive materials (64) of the second layer (82), wherein the conductive materials (64) of the first layer (80) and the conductive materials (64) of the second layer (82) are patterned in a direction substantially perpendicular to the axis of the lead system such that each of the conductive materials (64) of the first layer (80) is electrically isolated from the conductive materials (64) in the second layer (82).

2. A lead system as claimed in claim 1, wherein said conductive material (64) is a metal to shield said lead from electromagnetic interference.

3. A lead system as claimed in claim 1, wherein said conductive material (64) includes metal-coated carbon filaments to shield said lead from electromagnetic interference.

4. A lead system as claimed in claim 1, wherein said conductive material (64) is a polymer composite to shield said lead from electromagnetic interference.

5. A lead system as claimed in any preceding claim, wherein said shielding is constructed of two patterned sheaths, each patterned sheath having non-conductive materials (66) and conductive materials (64).

6. A lead system as claimed in claim 5, wherein said non-conductive materials (66) on one of said first and second patterned sheaths are randomly located.

7. A lead system as claimed in claim 5, wherein said non-conductive materials (66) in said first and second patterned sheaths are located in a predetermined segmented pattern.

**Patentansprüche**

1. Leitungssystem, das enthält:

eine Leitung (60), um einen elektrischen Weg zu einem Gewebebereich zu schaffen; und eine Abschirmung, die um die Leitung gebildet ist, um die Leitung von störender elektromagnetischer Einwirkung abzuschirmen, **dadurch gekennzeichnet, dass**

die Abschirmung aus einer ersten Schicht (80), die mit nicht leitenden Materialien (66) und leitendem Material (64) strukturiert ist, und einer zweiten Schicht (82), die mit nicht leitenden Materialien und leitenden Materialien strukturiert ist, gebildet ist;

wobei die leitenden Materialien (64) der ersten Schicht (80) in einer zu einer Achse des Leitungssystems im Wesentlichen parallelen Richtung strukturiert sind, so dass jedes der leitenden Materialien (64) von den anderen leitenden Materialien (64) der ersten Schicht (80) physikalisch getrennt ist;

wobei die leitenden Materialien (64) der zweiten Schicht (82) in einer zu einer Achse des Leitungssystems im Wesentlichen parallelen Achse strukturiert sind, so dass jedes der leitenden Materialien (64) von den anderen leitenden Materialien (64) der zweiten Schicht (82) physikalisch getrennt ist,

wobei die leitenden Materialien (64) der ersten Schicht (80) und die leitenden Materialien der zweiten Schicht (82) in einer zu einer Achse des Leitungssystems im Wesentlichen senkrechten Richtung strukturiert bzw. gemustert sind, so dass jedes der leitenden Materialien (64) der ersten Schicht (80) von den leitenden Materialien (64) der zweiten Schicht (80) elektrisch isoliert ist.

2. Leitungssystem nach Anspruch 1, wobei das leitende Material (64) ein Metall ist, um die Leitung von störender elektromagnetischer Einwirkung abzuschirmen.

3. Leitungssystem nach Anspruch 1, wobei das leitende Material (64) metallbeschichtete Kohlenstofffäden enthält, um die Leitung von störender elektromagnetischer Einwirkung abzuschirmen.

4. Leitungssystem nach Anspruch 1, wobei das leitende Material (64) eine Polymerzusammensetzung ist, um die Leitung von störender elektromagnetischer Einwirkung abzuschirmen.

5. Leitungssystem nach einem der vorhergehenden Ansprüche, wobei die Abschirmung aus zwei strukturierten Ummantelungen hergestellt ist, wobei jede strukturierte Ummantelung nicht leitende Materialien (66) und leitende Materialien (64) aufweist.

6. Leitungssystem nach Anspruch 5, wobei die nicht leitenden Materialien (66) auf der ersten oder der zweiten strukturierten Ummantelung zufällig angeordnet sind.

7. Leitungssystem nach Anspruch 5, wobei die nicht leitenden Materialien (66) in der ersten und der zweiten strukturierten Ummantelung in einem vorgegebenen segmentierten Muster angeordnet sind.


**Revendications**

1. Système de fil de dérivation, comportant :

un fil de dérivation (60) pour fournir un trajet électrique à une région tissulaire ; et
un blindage formé autour dudit fil de dérivation pour protéger ledit fil de dérivation vis-à-vis d'interférence électromagnétique ; **caractérisé en ce que** :

ledit blindage étant formé d'une première couche (80) modelée à l'aide de matériaux non conducteurs (66) et d'un matériau conducteur (64), et d'une seconde couche (82) modelée à l'aide de matériaux non conducteurs et de matériaux conducteurs ;
dans lequel les matériaux conducteurs (64) de la première couche (80) sont modelés dans une direction sensiblement parallèle à un axe du système de dérivation de sorte que chacun des matériaux conducteurs (64) est physiquement séparé des autres matériaux conducteurs (64) de la première couche (80) ;
dans lequel les matériaux conducteurs (64) de la seconde couche (82) sont modelés dans une direction sensiblement parallèle à un axe du système de fil de dérivation de sorte que chacun des matériaux conducteurs (64) est physiquement séparé des autres matériaux conducteurs (64) de la seconde couche (82),
dans lequel les matériaux conducteurs (64) de la première couche (80) et les matériaux conducteurs (64) de la seconde couche (82) sont modelés dans une direction sensiblement perpendiculaire à l'axe du système de fil de dérivation de sorte que chacun des matériaux conducteurs (64) de la première couche (80) est électriquement isolé des matériaux conducteurs (64) de la seconde couche (82).

2. Système de fil de dérivation comme revendiqué dans la revendication 1, dans lequel ledit matériau conducteur (64) est un métal pour protéger ledit fil de dérivation vis-à-vis d'interférence électromagnétique.

3. Système de fil de dérivation comme revendiqué dans la revendication 1, dans lequel ledit matériau con-

**EP 1 622 677 B1**

ducteur (64) inclut des filaments de carbone enduits de métal pour protéger ledit fil de dérivation vis-à-vis d'interférence électromagnétique.

4. Système de fil de dérivation comme revendiqué dans la revendication 1, dans lequel ledit matériau conducteur (64) est un composite de polymère pour protéger ledit fil de dérivation vis-à-vis d'interférence électronique.

5. Système de fil de dérivation comme revendiqué dans l'une quelconque des revendications précédentes, dans lequel ledit blindage est constitué de deux gaines modelées, chaque gaine modelée ayant des matériaux non conducteurs (66) et des matériaux conducteurs (64).

6. Système de fil de dérivation comme revendiqué dans la revendication 5, dans lequel lesdits matériaux non conducteurs (66) sur l'une desdites première et seconde gaines modelées sont positionnés de manière aléatoire.

7. Système de fil de dérivation comme revendiqué dans la revendication 5, dans lequel lesdits matériaux non conducteurs (66) dans lesdites première et seconde gaines modelées sont positionnés dans un modèle segmenté prédéterminé.

## FIG. 1

FIG. 2

FIG. 3

EP 1 622 677 B1

**FIG. 4**

EP 1 622 677 B1

**FIG. 5**

FIG. 6

EP 1 622 677 B1

FIG. 7

FIG. 8

FIG. 9

FIG. 10

EP 1 622 677 B1

FIG. 11

EP 1 622 677 B1

150 →

156

ECG AMPLIFIER & PROCESSOR

162 164 166 164 160 155 157 159

ELECTRODES — INDUCTORS — INPUT/OUTPUT RF FILTER — INITIAL AMPLIFIER — LOW - PASS FILTER — PROCESSOR

159 163 165

ELECTRODES — INDUCTORS

161 169 163 158 154

ELECTRODES — INDUCTORS — PACING OUTPUT STAGE — PACING LOGIC

167 167

152

EXTERNAL PACEMAKER PROGRAMMER

170

174 172 176

TELEMETRY RECEIVER/ BAND-PASS FILTER — TELEMETRY RF AMPLIFIER — TELEMETRY ENABLE LOGIC

168

**FIG. 12**

*178*

**FIG. 13**

*180*

**FIG. 14**

*182*    *184*

**FIG. 15**

**FIG. 16**

**FIG. 17**

**FIG. 18**

FIG. 19

FIG. 20

EP 1 622 677 B1

FIG. 21

FIG. 22

FIG. 23

EP 1 622 677 B1

FIG. 24

FIG. 25

FIG. 26

EP 1 622 677 B1

FIG. 27

FIG. 28

EP 1 622 677 B1

FIG. 29

FIG. 30

FIG. 31

FIG. 32

EP 1 622 677 B1

FIG. 33

FIG. 34

FIG. 35

FIG. 36

EP 1 622 677 B1

**EP 1 622 677 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5968083 A, Ciciarelli
- US 6188926 B, Vock
- US 5523534 A
- US 5827997 A
- US 5711300 A
- US 6396266 B
- US 4760336 A